# EUROPEAN PATENT SPECIFICATION

(11) **EP 3 497 449 B1**
(45) Date of publication and mention of the grant of the patent: **21.08.2024**
(21) Application number: 17839927.5
(22) Date of filing: 09.08.2017
(51) Int. Cl.: G01N 33/574, C07K 16/40, C12Q 1/48, G01N 33/573

(54) **DETERMINATION OF NON-HUMAN MAMMAL TK1 PROTEIN LEVELS**
BESTIMMUNG DER TK1-PROTEIN-SPIEGEL VON NICHTMENSCHLICHEN SÄUGETIEREN
DÉTERMINATION DE TENEUR EN PROTÉINES TK1 DE MAMMIFÈRE NON HUMAIN

(30) Priority: 10.08.2016 US 201662373262 P
(43) Date of publication of application: 19.06.2019
(73) Proprietor: Alertix Veterinary Diagnostics AB, 111 93 Stockholm (SE)
(72) Inventor: ERIKSSON, Staffan, 181 46 Lidingö (SE); RÖNNBERG, Henrik, 752 40 Uppsala (SE); JAGARLAMUDI, Kiran Kumar, 754 21 Uppsala (SE)
(74) Representative: Barker Brettell Sweden AB
(86) International application number: PCT/SE2017/050806
(87) International publication number: WO 2018/030946

(56) References cited:
- EP-B1- 1 627 230
- WO-A1-2015/094106
- US-A1- 2010 266 495
- LENA CARLSSON ET AL: "Elevated levels of thymidine kinase 1 peptide in serum from patients with breast cancer", UPSALA JOURNAL OF MEDICAL SCIENCES., vol. 114, no. 2, 1 January 2009 (2009-01-01), SE, pages 116 - 120, XP055667494, ISSN: 0300-9734, DOI: 10.1080/03009730802688835
- JAGARLAMUDI ET AL.: "A new sandwich ELISA for quantification of thymidine kinase 1 protein levels in sera from dogs with different malignancies can aid in disease managemen t", PLOS ONE, vol. 10, no. 9, 2015, pages e0137871/1 - e0137871/15, XP055464021
- JAGARLAMUDI ET AL.: "Properties of cellular and serum forms of thymidine kinase 1 (TK1) in dogs with acute lymphocytic leukemia (ALL) and canine mammary tumors (CMTs): implications for TK1 as a proliferation biomarker", BMC VETERINARY RESEARCH, vol. 10, 2014, pages 22 8/1 - 228/25, XP021200275

## Description

### TECHNICAL FIELD

The disclosure relates to determination of TK1 protein levels, and in particular to a kit and methods involving the use of anti-TK1 antibodies for determining canine, feline or equine TK1 protein levels.

### BACKGROUND

Dogs are frequently affected with various neoplastic diseases like lymphomas, leukemia's and mammary tumors. Lymphomas are the most common form of hematological tumors and accounts for 5 % of all cancers in dog. The annual incidence has been estimated to 13 to 40 cases per 100,000 dogs. Canine lymphoma is similar to human non-Hodgkin's lymphoma in terms of genetic and environmental factors that contribute to disease progression. Early stage diagnosis in combination with effective chemotherapy can control the malignancy. Several proliferation markers including argyrophilic nucleolar organizing regions (AgNORs), proliferation cell nuclear antigen (PCNA) and Ki-67 have been investigated as prognostic markers in canine lymphoma, but their usage is limited to immunohistochemistry. Serum lactate dehydrogenase (LDH) was also investigated as a marker for monitoring canine lymphomas, but LDH is up regulated in diseases other than malignancies and thus has a limited clinical value (von Euler et al., 2006).

Also other non-human mammals are affected with various neoplastic diseases, such as cats and horses. Tumor progression is dependent on cell proliferation and proliferation markers are valuable in order to detect tumor diseases at an early stage. Thymidine kinase 1 (TK1) is one of the biomarkers that is released into the blood during uncontrolled cell growth. TK1 converts deoxythymidine (dT) to deoxythymidine monophosphate (dTMP), which is eventually incorporated into DNA in proliferating cells. TK1 activity is tightly associated with the cell cycle and reaches a peak in S-phase, declines rapidly in G2, and is degraded by specific mechanisms in M phase.

Serum TK1 activity measurements is an established tool for diagnosis and monitoring of lymphomas and leukemia's in human medicine. Serum TK1 activity is measured by using several enzymatic assays e.g., TK radioenzymatic assay (TK-RIA), such as PROLIFIGENO, or TK chemiluminescent immune assay (TK-CLIA), such as LIAISON^{®} TK, where a thymidine substrate analogue, like ¹²⁵I-iododeoxyuridine or azidothymidine (AZT), is phosphorylated into the corresponding monophosphate. Studies have shown that both TK-REA and TK-CLIA assays provide valuable information for prognosis and treatment monitoring of canine hematological tumors. A recent study, using the natural substrate [³H]-dThd (deoxy thymidine) instead of substrate analogues, showed that this assay was equally sensitive as the TK-REA and TK-CLIA assays and could be used for monitoring canine lymphomas (Sharif et al., 2012 a).

The development of antibodies against different regions of the human TK1 protein has extended the clinical utility of TK1 determinations further. Several clinical studies based on antibody detection methods demonstrated increased TK1 protein levels in sera from patients with various tumor diseases. Furthermore, it was found that the TK1 protein assays were more sensitive than the TK1 activity assays for prognosis and treatment monitoring of solid tumors. Several attempts have been made to develop TK Enzyme-Linked Immunosorbent Assay (TK-ELISA) methods for detection of breast cancer, hematologic malignancies and lung cancer in human medicine (Carlsson et al., 2009; Alegre et al., 2014).

Presently, there are no immunochemical methods available for non-human mammal oncology studies. However, recently studies have been performed using an immunoaffinity assay based on antibodies against the dog TK1 C-terminal (Kiran Kumar et al., 2013; Jagarlamudi et al., 2014). This allowed determination of serum TK1 protein levels in canine subjects with various malignancies and concluded that the TK1 protein assay was more sensitive than TK1 activity assay for differentiation of canine solid tumors from healthy dogs. Kiran Kumar 2010 disclosed production of anti-dog TK1 antibodies for detection of serum TK1. The antibodies were produced by immunizing rabbits with a 28 amino acid long peptide corresponding to amino acids 196 to 223 in dog TK1.

Carlsson et al., "Elevated levels of thymidine kinase 1 peptide in serum from patients with breast cancer", Upsala Journal of Medical Science, 114(2): 116-120 (2009) discloses evaluation of a new immunological sandwich assay for detection of TK peptides in serum from breast cancer patients.

WO 2015/094106 discloses monoclonal antibodies or fragments capable of binding to a serum form of human TK1 and to kits and methods involving the use of such monoclonal antibodies or fragments.

Jagarlamudi et al., "A new sandwich ELISA for quantification of thymidine kinase 1 protein levels in sera from dogs with different malignancies can aid in disease management", PLOS ONE, 10(9): E0137871/1 - 15 (2015) discloses the development of a specific sandwich TK1-ELISA for the quantification of TK1 protein levels in sera from dogs with different malignancies.

Jagarlamudi et al., "Properties of cellular and serum forms of thymidine kinase 1 (TK1) in dogs with acute lymphocytic leukemia (ALL) and canine mammary tumors (CMTs): implications for TK1 as a proliferation biomarker", BMC Veterminary Research 10: 228 discloses that serum TK1 protein and activity levels were significantly higher in CMT than in healthy dogs. Size exclusion chromatography demonstrated major differences in the molecular forms of sTK1 in ALL, healthy, and CMT dogs, with a large fraction of inactive TK1 protein in CMT.

US 2010/266495 discloses methods of diagnosing and treating cancers that overexpress thymidine kinase 1 on their cell surfaces. These methods utilize monoclonal antibodies that bind specifically to thymidine kinase 1 to inhibit or prevent proliferation of the cancer cells.

EP 1627230 discloses early prediction of progression of a cancer disease in patients treated for cancer. The binding response level of an immunoreactive material (IM) comprising thymidine kinase 1 (TK1) protein is determined in a body fluid sample of a patient and the likelihood of progress of the cancer disease is estimated based directly on this determined IM binding response level. Those patients that run a risk of cancer progress and tumor relapse 1-15 years after the cancer treatment may be identified directly by the IM binding response level according to the invention within a few months following initiation of the treatment.

There is, however, still a need for a clinically acceptable technique for measuring TK1 protein levels in non-human mammal subjects.

### SUMMARY

It is a general objective to provide a kit and methods for measuring TK1 protein levels dogs, cats or horses.

The present invention is defined in the independent claims. Further embodiments of the invention are defined in the dependent claims.

An aspect of the embodiments relates to a kit according to claim 1 for determining a level of non-human mammal TK1 protein in a sample. The kit comprises a first antibody immobilized to a support or intended to be immobilized to the support and a second antibody. One of the first antibody and the second antibody has specificity for a peptide consisting of an amino acid sequence from a C-terminal region of non-human mammal TK1. The other of the first antibody and second antibody has specificity for a peptide consisting of a first amino acid sequence from an active site of TK1. The non-human mammal TK1 is selected from the group consisting of canine TK1 protein, feline TK1 protein and equine TK1 protein. The one of the first antibody and the second antibody has specificity for a peptide consisting of an amino acid sequence of SEQ ID NO: 11 and the other of the first antibody and second antibody has specificity for a peptide consisting of an amino acid sequence of SEQ ID NO: 2.

Another aspect of the embodiments relates to an *in vitro* method for determining a level of canine, feline or equine TK1 protein in a sample according to the claims. The method comprises contacting the sample with a first antibody and a second antibody of the kit as defined above. The method also comprises detecting an amount of bound second antibody.

A further aspect of the embodiments relates to an *in vitro* method for estimating the likelihood of recurrence of a tumor disease in a canine, feline or equine subject according to the claims. The method comprises determining a level of canine, feline or equine TK1 protein in a body sample from the canine, feline or equine subject using the *in vitro* a method or the kit according to above. The method also comprises comparing the level of canine, feline or equine TK1 protein in the body sample with a level of canine, feline or equine TK1 protein representative of a population of healthy canine, feline or equine subjects or with a level of canine, feline or equine TK1 protein previously determined in the canine, feline or equine subject. The method further comprises estimating the likelihood of recurrence of the tumor disease in the non-human mammal subject based on the comparison.

Yet another aspect of the embodiments relates to an *in vitro* method for determining cell proliferation in a canine, feline or equine subject according to the claims. The method comprises determining a level of canine, feline or equine TK1 protein in a body sample from the canine, feline or equine subject using the *in vitro* a method or the kit according to above. The method also comprises determining the cell proliferation based on the level of canine, feline or equine TK1 protein in the body sample.

A further aspect of the embodiments relates to an *in vitro* method for determining a proliferation process response in a canine, feline or equine subject suffering from a malignant disease according to the claims. The method comprises determining a level of canine, feline or equine TK1 protein in a body sample from the canine, feline or equine subject using the *in vitro* a method or the kit according to above. The method also comprises determining the proliferation process response based on the level of canine, feline or equine TK1 protein in the body sample.

Yet another aspect of the embodiments relates to a method for determining a level of inflammation, infection, or tumor cell proliferation in a canine, feline or equine subject according to the claims. The method comprises determining a level of canine, feline or equine TK1 protein in a body sample from the canine, feline or equine subject using the *in vitro* a method or the kit according to above. The method also comprises determining the level of inflammation, infection or tumor cell proliferation based on the level of canine, feline or equine TK1 protein in the body sample.

A further aspect of the embodiments relates to an *in vitro* method for evaluating efficiency of a treatment of a malignant disease in a canine, feline or equine subject according to the claims. The method comprises determining a level of canine, feline or equine TK1 protein in a body sample from the canine, feline or equine subject using a method or a kit according to the embodiments prior to or in connection with start of the treatment of the malignant disease. The method also comprises determining a level of canine, feline or equine TK1 protein in a body sample from the canine, feline or equine subject using the *in vitro* method or kit according to the embodiments during or after the treatment of the malignant disease. The method further comprises evaluating efficiency of the treatment of the malignant disease based on a comparison of the level of canine, feline or equine TK1 protein determined in the body sample prior to or in connection with start of the treatment of the malignant disease and the level of canine, feline or equine TK1 protein determined in the body sample during or after the treatment of the malignant disease.

The embodiments enable determining TK1 protein levels in canine, feline or equine subject. The kit and methods of the embodiments are easy to perform, fast and as sensitive and specific as existing TK1 activity assays. The kit and methods are furthermore far more sensitive than TK1 activity assays in differentiating healthy dogs, cats or horses from dogs, cats or horses suffering from solid tumors.

### BRIEF DESCRIPTION OF THE DRAWINGS

The embodiments, together with further objects and advantages thereof, may best be understood by making reference to the following description taken together with the accompanying drawings, in which:

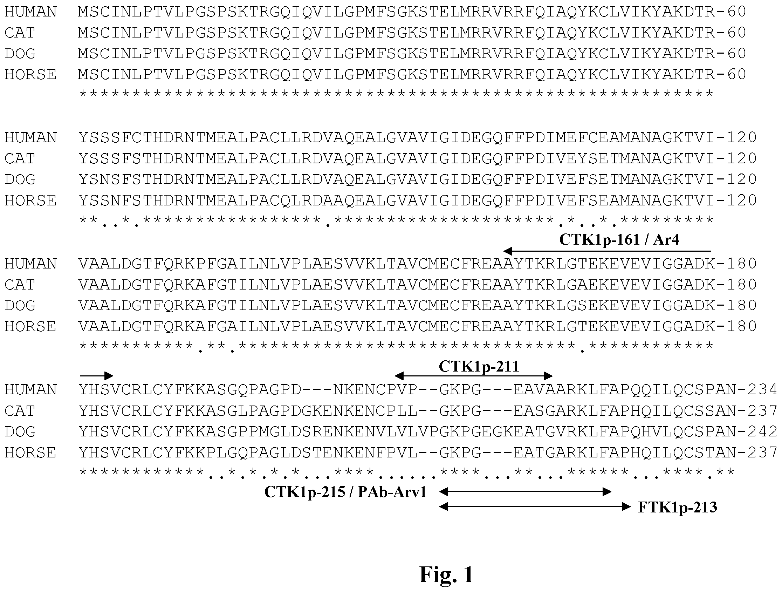

Fig. 1 illustrates amino acid sequence alignments of human TK1 (GenBank accession no. KO_2582; SEQ ID NO: 4), dog TK1 (also referred to as canine TK1 herein, GenBank accession no. XM_540461; SEQ ID NO: 3), cat TK1 (also referred to as feline TK1 herein, GenBank accession no. XP_3997286.2; SEQ ID NO: 9) and horse TK1 (also referred to as equine TK1 herein, GenBank accession no. XP_1491131.2; SEQ ID NO: 17) deduced amino acid sequences. In horse TK1, an addition of an oligonucleotide sequence coding for the conserved N-terminal peptide (amino acids 1-27 in TK1 in human, dog and cat TK1) MSCINLPTVLPGSPSKTRGQIQVILGP (SEQ ID NO: 18) was made to make the GenBank deduced cDNA sequence in order to produce a recombinant horse TK1 protein with similar overall length as the dog TK1 and cat TK1 proteins, see SEQ ID NO: 10. Antibodies raised against the active site region of dog TK1 (CTK1p-161, amino acids 161-183 in dog TK1, AYTKRLGSEKEVEVIGGADKYHS (SEQ ID NO: 7)), against the C-terminal region of dog TK1 (CTK1p-211, amino acids 211-225 in dog TK1, VLVPGKPGEGKEATG (SEQ ID NO: 11); CTK1p-215, amino acids 215-230 in dog TK1, GKPGEGKEATGVRKLF (SEQ ID NO: 1)) and against the C-terminal region of cat TK1 (FTK1p-213, amino acids 213-227 in cat TK1, GKPGEASGARKLFAP (SEQ ID NO: 13)). The figure also shows mouse monoclonal anti-TK1 antibodies raised against the active site region of human TK1 (Ar-4, amino acids 161-183 in human TK1, AYTKRLGTEKEVEVIGGADKYHS (SEQ ID NO: 2)) and rabbit polyclonal antibodies against the C-terminal region of dog TK1 (PAb-Arv1, amino acids 215-230 in dog TK1, GKPGEGKEATGVRKLF (SEQ ID NO: 1)).

Fig. 2 is a schematic presentation of a sandwich ELISA. The five steps are: (1) coating of microplate wells with a first anti-TK1 antibody (capture or coating antibody), (2) binding of TK1 in serum to the capture antibody, (3) attachment of a biotinylated anti-TK1 antibody (detection antibody) to TK1 in serum, (4) detection of biotin by streptavidin-HRP, and (5) enzymatic activity monitored by addition of 3,3-,5,5-tetramethylbenzidine (TMB) chromogenic substrate.
Fig. 3 illustrates a standard curve with different concentrations ranging from 0.6 to 10 ng/mL of recombinant dog TK1 against the absorbance at 450 nM from 5 different runs.
Fig. 4A illustrates log STK1 activity levels (pmol/min/mL) in sera from healthy dogs (•) and dogs with hematological tumors (▪). The straight lines represent the median value.
Fig. 4B illustrates receiver operating characteristic (ROC) curve of STK1 activity assay results from dogs with hematological tumors (n=36) and from healthy dog (n=30).
Fig. 4C illustrates log STK1 protein levels in sera from healthy dogs (•) and dogs with hematological tumors (▪). The straight lines represent the median value.
Fig. 4D illustrates ROC curve analysis for the STK1 protein levels (ng/mL) of healthy dogs in comparison to those in dogs with hematological tumors.
Fig. 5A illustrates log STK1 activity levels (pmol/min/mL) in sera from healthy dogs (•) and dogs with solid tumors (▪). The straight lines represent the median value.
Fig. 5B illustrates ROC curve of STK1 activity assay results from dogs with solid tumors (n=40) and from healthy dogs (n=30).
Fig. 5C illustrates log STK1 protein levels in sera from healthy dogs (•) and dogs with solid tumors (▪). The straight lines represent the median value.
Fig. 5D illustrates ROC curve analysis for the STK1 protein levels (ng/mL) of healthy dogs in comparison to those in dogs with solid tumors.
Fig. 6 illustrates log STK1 distribution in different sub groups of dog tumors. (A) Log STK1 activity levels in sera from healthy dogs, dogs with lymphoma, dogs with leukemia. (B) Log STK1 activity levels in healthy dogs, dogs with mammary tumors, dogs with malignant melanomas, dogs with mastocytomas and dogs with other tumors. (C) STK1 protein distribution in sera from healthy dogs, dogs with lymphoma, dogs with leukemia. (D) STK1 protein distribution in healthy dogs, dogs with mammary tumors, dogs with malignant melanomas, dogs with mastocytomas and dogs with other tumors
Fig. 7A illustrates STK1 activity levels in sera from dogs during chemotherapy of dogs with lymphoma.
Fig. 7B illustrates STK1 protein levels in sera from dogs during chemotherapy of dogs with lymphoma.
Fig. 8 illustrates results of a dot blot immunoassay with dog, cat and horse recombinant TK1 with (A) CTK1p-215 antiserum, (B) CTK1p-161 antiserum, (C) CTK1p-211 antiserum and (D) FTK1p-213 antiserum.
Fig. 9 illustrates dot intensities in arbitrary units (A.U.) with different concentrations of (A) dog recombinant TK1, (B) cat recombinant TK1 and (C) horse recombinant TK1 with different antisera.
Fig. 10 illustrates a standard curve with different concentrations ranging from 0.6 to 10 ng/mL of recombinant dog TK1 in five different experiments with CTK1p-215 coating and CTK1p-161 as detection antibodies.
Fig. 11A illustrates log STK1 ELISA protein levels (ng/mL) in sera from healthy dogs (•) and dogs with hematological malignancies (▪). The error bars represent median.
Fig. 11B illustrates ROC curve of STK1 ELISA assay for discrimination of hematological tumor dogs (n=15) from healthy (n=10).
Fig. 11C illustrates log STK1 protein levels in sera from healthy dogs (•) and dogs with solid tumors (▪). The error bars represent median.
Fig. 11D illustrates ROC curve analysis for the STK1 protein levels (ng/mL) of healthy in comparison to those in dogs with solid tumors (n=10).
Fig. 12 illustrates a standard curve with different concentrations ranging from 0.6 to 10 ng/mL of recombinant dog TK1 against the absorbance at 450 nM from five different runs with CTK1p-211 coating and CTK1p-215 detection antibodies.
Fig 13A illustrates log STK1 protein distribution (ng/mL) in sera from healthy dogs (•) and dogs with hematological tumors (▪). The error bars represent median.
Fig. 13B illustrates ROC curve of STK1 ELISA assay for discrimination of hematological tumors (n=15) from healthy (n=7).

### DETAILED DESCRIPTION

The present disclosure relates to determination of canine, feline or equine TK1 protein levels, and in particular to a kit and methods involving the use of anti-TK1 antibodies for determining canine, feline or equine TK1 protein levels.

Reference to non-human mammalian TK1 protein level and non-human mammalian subject herein are reference to canine (dog), feline (cat) or equine (horse) TK1 protein level and canine, feline or equine subject.

The crystal structure of human TK1 has been resolved and the major part of the enzyme, including the N-terminal region, is conserved in humans, dogs, cats and horses except for a few residues, see Fig. 1 and SEQ ID NO: 3 representing dog TK1 (canine TK1), SEQ ID NO: 4 representing human TK1, SEQ ID NO: 9 representing cat TK1 (feline TK1) and SEQ ID NO: 10 representing horse TK1 (equine TK1). For instance, human TK1 sequence shows 88.5 % similarity with dog TK1 but a significant sequence diversity (9.1 %) is found in the C-terminal region.

Disclosed is a kit for determining a level of non-human mammal TK1 protein in a sample. The kit comprises a first antibody immobilized to a support or intended to be immobilized to the support and a second antibody. One of the first antibody and the second antibody has specificity for a peptide consisting of an amino acid sequence from a C-terminal region of non-human mammal TK1. The other of the first antibody and second antibody has specificity for a peptide consisting of a first amino acid sequence from an active site of TK1. The non-human mammal TK1 is selected from a group consisting of canine TK1 protein, feline TK1 protein and equine TK1 protein.

The kit is in particular suitable for determining the level of non-human mammal serum TK1 (STK1) protein in sample, preferably a body sample from a non-human mammal subject.

The peptide consisting of a first amino acid sequence or a second amino acid sequence from the C-terminal regional is preferably a peptide selected from a portion of the non-human mammal TK1 ranging from amino acid position 200 to the end of the non-human mammal TK1 (amino acid position 242 in dog, amino acid position 237 in cat and amino acid position 237 in horse). In particular, the peptide is selected from a portion of the TK1 protein ranging from amino acid position 205, preferably 210 and more preferably 211, to amino acid position 240, preferably 235 and more preferably 230. The peptide is preferably an N-mer, wherein N is an integer within a range of 10 and 25, preferably 10 to 20 and more preferably 15, 16 or 17.

The peptide preferably consists of N consecutive amino acids in the C-terminal region of the non-human mammal TK1 protein.

At least one additional amino acid, such as a cysteine residue, may be added to the N-terminal or C-terminal, preferably the N-terminal, of the peptide for use as coupling to other molecules, such as carrier proteins.

In an example not part of the invention, the peptide consisting of a first amino acid sequence or a second amino acid sequence from the C-terminal region has an amino acid sequence corresponding to amino acid positions 215 to 230 in canine TK1, see Fig. 1, i.e., has amino acid sequence of GKPGEGKEATGVRKLF (SEQ ID NO: 1). The corresponding amino acid sequence with the added N-terminal cysteine residue is CGKPGEGKEATGVRKLF (SEQ ID NO: 5).

According to the invention, the peptide consisting of a first amino acid sequence or a second amino acid sequence from the C-terminal region has an amino acid sequence corresponding to amino acid positions 211 to 225 in canine TK1, see Fig. 1, i.e., has amino acid sequence of VLVPGKPGEGKEATG (SEQ ID NO: 11). The corresponding amino acid sequence with the added N-terminal cysteine residue is CVLVPGKPGEGKEATG (SEQ ID NO: 12).

In another example not part of the invention, the peptide consisting of a first amino acid sequence or a second amino acid sequence from the C-terminal region has an amino acid sequence corresponding to amino acid positions 213 to 227 in feline TK1, see Fig. 1, i.e., has amino acid sequence of GKPGEASGARKLFAP (SEQ ID NO: 13). The corresponding amino acid sequence with the added N-terminal cysteine residue is CGKPGEASGARKLFAP (SEQ ID NO: 14).

The peptide consisting of an amino acid sequence from an active site of TK1 is preferably a peptide selected from a portion of TK1 ranging from amino acid position 150 to amino acid position 190. In particular, the peptide is selected from a portion of TK1 ranging from amino acid position 155, preferably 160 and more preferably 161, to amino acid position 185, preferably 183.

The peptide is preferably an M-mer, wherein M is an integer within a range of 10 and 40, preferably 20 to 30 and more preferably 23 or 24.

The peptide preferably consists of M consecutive amino acids in the active site of the TK1 protein.

This portion representing the active site of TK1 shows very high levels of homology between different species, such as between human, canine, feline and equine TK1 as shown in Fig. 1. For instance, there is only a difference in a single amino acid position between human TK1 and canine TK1 and feline TK1 within the portion extending from amino acid position 150 to amino acid position 190. There are no amino acid differences in this region between human TK1 and equine TK1.

Hence, the peptide could consist of an amino acid sequence from an active site of human TK1, from canine TK1, from feline TK1, from equine TK1 or from another non-human mammal TK1. Experimental data as presented herein shows that an antibody that has specificity for a peptide consisting of an amino acid sequence from the active site of human TK1 binds specifically also to canine TK1.

At least one additional amino acid, such as a cysteine residue, may be added to the N-terminal or C-terminal, preferably the N-terminal, of the peptide for use as coupling to other molecules, such as carrier proteins.

According to the invention, the peptide consisting of an amino acid sequence from the active site of TK1 has an amino acid sequence corresponding to amino acid positions 161 to 183 in human TK1 (and equine TK1), see Fig. 1, i.e., has amino acid sequence of AYTKRLGTEKEVEVIGGADKYHS (SEQ ID NO: 2). The corresponding amino acid sequence with the added N-terminal cysteine residue is CAYTKRLGTEKEVEVIGGADKYHS (SEQ ID NO: 6).

In an example not part of the invention, the peptide consisting of an amino acid sequence from the active site of TK1 has an amino acid sequence corresponding to amino acid positions 161 to 183 in canine TK1, see Fig. 1, i.e., has amino acid sequence of AYTKRLGSEKEVEVIGGADKYHS (SEQ ID NO: 7). The corresponding amino acid sequence with the added N-terminal cysteine residue is CAYTKRLGSEKEVEVIGGADKYHS (SEQ ID NO: 8).

In another example not part of the invention, the peptide consisting of an amino acid sequence from the active site of TK1 has an amino acid sequence corresponding to amino acid positions 161 to 183 in feline TK1, see Fig. 1, i.e., has amino acid sequence of AYTKRLGAEKEVEVIGGADKYHS (SEQ ID NO: 15). The corresponding amino acid sequence with the added N-terminal cysteine residue is CAYTKRLGAEKEVEVIGGADKYHS (SEQ ID NO: 16).

A kit for determining the level of non-human mammal TK1 protein in a sample comprises the first antibody immobilized to a support or intended to be immobilized to the support and the second antibody. In this embodiment, one of the first and the second antibody has specificity for a peptide consisting of an amino acid sequence from the active site of TK1 and the other of the first antibody and the second antibody has specificity for a peptide consisting of an amino acid sequence from the C-terminal region of TK1.

In a particular example not part of the invention, one of the first antibody and the second antibody has specificity for a peptide consisting of an amino acid sequence selected from a group consisting of SEQ ID NO: 11 and SEQ ID NO: 12 and the other of the first antibody and the second antibody has specificity for a peptide consisting of an amino acid sequence selected from a group consisting of SEQ ID NO: 2, SEQ ID NO: 6, SEQ ID NO: 7 and SEQ ID NO: 8. The combination of the first and second antibodies according to the invention, thus, involves an antibody having specificity for a peptide consisting of an amino acid sequence consisting of SEQ ID NO: 11 and an antibody having specificity for a peptide consisting of an amino acid sequence consisting of SEQ ID NO: 2. Particular combinations of the first and second antibodies according examples not part of the invention involve, an antibody having specificity for a peptide consisting of an amino acid sequence consisting of SEQ ID NO: 11 and an antibody having specificity for a peptide consisting of an amino acid sequence consisting of SEQ ID NO: 6, an antibody having specificity for a peptide consisting of an amino acid sequence consisting of SEQ ID NO: 11 and an antibody having specificity for a peptide consisting of an amino acid sequence consisting of SEQ ID NO: 7, an antibody having specificity for a peptide consisting of an amino acid sequence consisting of SEQ ID NO: 11 and an antibody having specificity for a peptide consisting of an amino acid sequence consisting of SEQ ID NO: 8, an antibody having specificity for a peptide consisting of an amino acid sequence consisting of SEQ ID NO: 12 and an antibody having specificity for a peptide consisting of an amino acid sequence consisting of SEQ ID NO: 2, an antibody having specificity for a peptide consisting of an amino acid sequence consisting of SEQ ID NO: 12 and an antibody having specificity for a peptide consisting of an amino acid sequence consisting of SEQ ID NO: 6, an antibody having specificity for a peptide consisting of an amino acid sequence consisting of SEQ ID NO: 12 and an antibody having specificity for a peptide consisting of an amino acid sequence consisting of SEQ ID NO: 7, and an antibody having specificity for a peptide consisting of an amino acid sequence consisting of SEQ ID NO: 12 and an antibody having specificity for a peptide consisting of an amino acid sequence consisting of SEQ ID NO: 8.

According to the invention, one of the first antibody and the second antibody is the CTK1p-211 antibody and the other of the first antibody and the second antibody is the CTK1p-161 antibody. In an example not part of the invention, one of the first antibody and the second antibody is the CTK1p-211 antibody and the other of the first antibody and the second antibody is the Arv4 antibody.

In another particular example not part of the invention, one of the first antibody and the second antibody has specificity for a peptide consisting of an amino acid sequence selected from a group consisting of SEQ ID NO: 1 and SEQ ID NO: 5 and the other of the first antibody and the second antibody has specificity for a peptide consisting of an amino acid sequence selected from a group consisting of SEQ ID NO: 2, SEQ ID NO: 6, SEQ ID NO: 7 and SEQ ID NO: 8. Particular combinations of the first and second antibodies according to this example not part of the invention, thus, involve an antibody having specificity for a peptide consisting of an amino acid sequence consisting of SEQ ID NO: 1 and an antibody having specificity for a peptide consisting of an amino acid sequence consisting of SEQ ID NO: 2, an antibody having specificity for a peptide consisting of an amino acid sequence consisting of SEQ ID NO: 1 and an antibody having specificity for a peptide consisting of an amino acid sequence consisting of SEQ ID NO: 6, an antibody having specificity for a peptide consisting of an amino acid sequence consisting of SEQ ID NO: 1 and an antibody having specificity for a peptide consisting of an amino acid sequence consisting of SEQ ID NO: 7, an antibody having specificity for a peptide consisting of an amino acid sequence consisting of SEQ ID NO: 1 and an antibody having specificity for a peptide consisting of an amino acid sequence consisting of SEQ ID NO: 8, an antibody having specificity for a peptide consisting of an amino acid sequence consisting of SEQ ID NO: 5 and an antibody having specificity for a peptide consisting of an amino acid sequence consisting of SEQ ID NO: 2, an antibody having specificity for a peptide consisting of an amino acid sequence consisting of SEQ ID NO: 5 and an antibody having specificity for a peptide consisting of an amino acid sequence consisting of SEQ ID NO: 6, an antibody having specificity for a peptide consisting of an amino acid sequence consisting of SEQ ID NO: 5 and an antibody having specificity for a peptide consisting of an amino acid sequence consisting of SEQ ID NO: 7, and an antibody having specificity for a peptide consisting of an amino acid sequence consisting of SEQ ID NO: 5 and an antibody having specificity for a peptide consisting of an amino acid sequence consisting of SEQ ID NO: 8.

In an example not part of the invention, one of the first antibody and the second antibody is the CTK1p-215 antibody and the other of the first antibody and the second antibody is the CTK1p-161 antibody. In another preferred embodiment, one of the first antibody and the second antibody is the CTK1p-215 antibody and the other of the first antibody and the second antibody is the Arv4 antibody. In another example not part of the invention, one of the first antibody and the second antibody is the PAb-Arv1 antibody and the other of the first antibody and the second antibody is the CTK1p-161 antibody. In yet another preferred embodiment, one of the first antibody and the second antibody is the PAb-Arv1 antibody and the other of the first antibody and the second antibody is the Arv4 antibody.

In an example not part of the invention, the kit for determining the level of non-human mammal TK1 protein in a sample comprises the first antibody immobilized to a support or intended to be immobilized to the support and the second antibody. In this example not part of the invention, one of the first and the second antibody has specificity for a peptide consisting of a first amino acid sequence from the C-terminal region of TK1 and the other of the first antibody and the second antibody has specificity for a peptide consisting of a second amino acid sequence from the C-terminal region of TK1.

The first and second amino acid sequences are different amino acid sequences from the C-terminal region of TK1. The two amino acid sequences may, however, be partially overlapping as shown in Fig. 1 when comparing, for instance, the amino acid sequence for CTK1p-211 with the amino acid sequence for CTK1p-215.

For instance, if the first amino acid sequence consists of N₁ amino acids and the second amino acid sequence consists of N₂ amino acids then the two amino acid sequences could be overlapping with P amino acids. P is equal to or larger than 0 but smaller than the largest of N₁ and N₂, i.e., 0 ≤ P < max( N₁, N₂).

It was highly surprising that two antibodies having specificity for different epitopes, which may be at least partly overlapping, in the C-terminal region of TK1 could be used to determine the level of non-human mammal TK1 protein. A possible reason for this surprising finding could be that TK1 is polyvalent and has several antibody binding sites both on recombinant TK1 and serum TK1. However, in spite of having multiple potential antibody binding sites in the recombinant and serum TK1 complexes it is generally preferred to use two different antibodies having specificity for different epitopes to reduce cross-reactivity and increase the specificity.

In a particular example not part of the invention, one of the first antibody and the second antibody has specificity for a peptide consisting of an amino acid sequence selected from a group consisting of SEQ ID NO: 11 and SEQ ID NO: 12 and the other of the first antibody and the second antibody has specificity for a peptide consisting of an amino acid sequence selected from a group consisting of SEQ ID NO: 1 and SEQ ID NO: 5. Particular combinations of the first and second antibodies according to this example not part of the invention, thus, involve an antibody having specificity for a peptide consisting of an amino acid sequence consisting of SEQ ID NO: 11 and an antibody having specificity for a peptide consisting of an amino acid sequence consisting of SEQ ID NO: 1, an antibody having specificity for a peptide consisting of an amino acid sequence consisting of SEQ ID NO: 11 and an antibody having specificity for a peptide consisting of an amino acid sequence consisting of SEQ ID NO: 5, an antibody having specificity for a peptide consisting of an amino acid sequence consisting of SEQ ID NO: 12 and an antibody having specificity for a peptide consisting of an amino acid sequence consisting of SEQ ID NO: 1 and an antibody having specificity for a peptide consisting of an amino acid sequence consisting of SEQ ID NO: 12 and an antibody having specificity for a peptide consisting of an amino acid sequence consisting of SEQ ID NO: 5.

In an example not part of the invention, one of the first antibody and the second antibody is the CTK1p-211 antibody and the other of the first antibody and the second antibody is the CTK1p-215 antibody. In another example not part of the invention, one of the first antibody and the second antibody is the CTK1p-211 antibody and the other of the first antibody and the second antibody is the PAb-Arv1 antibody. The antibodies may be polyclonal antibodies, monoclonal antibodies or one them may be a polyclonal antibody with the other as a monoclonal antibody.

The antibody that has specificity for the peptide consisting of an amino acid sequence from the C-terminal region of non-human mammal TK1 may be a polyclonal antibody and the antibody that has specificity for the peptide consisting of an amino acid sequence from the active site of TK1 may be a monoclonal antibody.

One or both of the antibodies may be an antibody fragment having specificity for the relevant peptide. In such a case, the fragment can be selected from a group consisting of a single chain antibody, a Fv fragment, a scFv fragment, a Fab fragment, a F(ab')2 fragment, a Fab' fragment, a Fd fragment, a singledomain antibody (sdAb), a scFv-Fc fragment, a di-scFv fragment and a CDR region.

The specificity of an antibody can be determined based on affinity and/or avidity. The affinity, represented by the equilibrium constant for the dissociation of an antigen with the antibody (*K*_{d}), is a measure for the binding strength between an antigenic determinant and an antigen-binding site on the antibody. The lesser the value of *K*_{d}, the stronger the binding strength between the antigenic determinant and the antibody. Alternatively, the affinity can also be expressed as the affinity constant (*K*ₐ), which is 1/*K*_{d}. As will be clear to the skilled person, affinity can be determined in a manner known per se, depending on the specific antigen of interest.

Avidity is the measure of the strength of binding between an antibody and the pertinent antigen. Avidity is related to both the affinity between an antigenic determinant and its antigen binding site on the antibody and the number of pertinent binding sites present on the monoclonal antibody.

Typically, antibodies will bind to their antigen with a dissociation constant (*K*_{d}) of 10⁻⁵ to 10⁻¹² moles/liter (M) or less, and preferably 10⁻⁷ to 10⁻¹² M or less and more preferably 10⁻⁸ to 10⁻¹² M, i.e. with an association constant (*K*ₐ) of 10⁵ to 10⁻¹² M⁻¹ or more, and preferably 10⁷ to 10⁻¹² M⁻¹ or more and more preferably 10⁸ to 10⁻¹² M⁻¹.

Generally, any *K*_{d} value greater than 10⁻⁴ M (or any Kₐ value lower than 10⁴ M⁻¹) is generally considered to indicate non-specific binding.

Preferably, an antibody or fragment will bind to the serum form and/or recombinant form of non-human mammal TK1 with an affinity less than 500 nM, preferably less than 200 nM, more preferably less than 10 nM, such as less than 5 nM.

In a particular embodiment, the kit is a sandwich assay kit. This means that the kit uses antibodies binding to different epitopes of non-human mammal TK1 protein so that both the first and second antibodies can simultaneously bind to the same non-human mammal TK1 molecule or complex.

In a particular embodiment, the kit is an Enzyme-Linked Immunosorbent Assay (ELISA) kit and preferably a sandwich ELISA.

A sandwich ELISA can be used to detect cellular and/or serum TK1 protein in a sample by preparing a surface of a support, such as a solid support, to which the first antibody is bound as so-called capture antibody. In a preferred embodiment, a known quantity of the first antibody is bound to the surface of the support. Any nonspecific binding sites on the surface are optionally but preferably blocked. The sample is then applied to the surface so that any non-human mammal TK1 protein present therein will be captured by the immobilized first antibodies. Unbound material is preferably removed by one or multiple washing steps. The second antibody, typically denoted detection antibody, is then added and is allowed to bind to any non-human mammal TK1 protein captured by the first antibody.

The amount of bound second antibody is then determined by direct or indirect detection methods. For instance, a label or enzyme can be attached directly to the second antibody or indirectly via a link, such as a biotin-streptavidin or a biotin-avidin link. It is, alternatively, possible to use a secondary antibody that is labeled or connected to an enzyme and binds specifically to the second antibody.

Hence, in an embodiment the second antibody has a covalently attached biotin. Alternatively, the second antibody has a covalently attached streptavidin or avidin.

The kit preferably also comprises a horseradish peroxidase (HRP) labeled streptavidin or a HRP labeled avidin. Alternatively, the kit also comprises a HRP labeled biotin. The kit also comprises a HRP substrate, such as a 3,3',5,5'-tetramethylbenzidine (TMB) substrate, a 3,3'-diaminobenzidine (DAB) substrate or a 2,2'-azino-bis(3-ethylbenzothiazoline-6-sulphonic acid (ABTS) substrate. In such a case, the level of non-human mammal TK1 protein in the sample can be determined by spectrophotometric methods that detect the conversion of the chromogenic substrate by HRP into a colored product that is detectable.

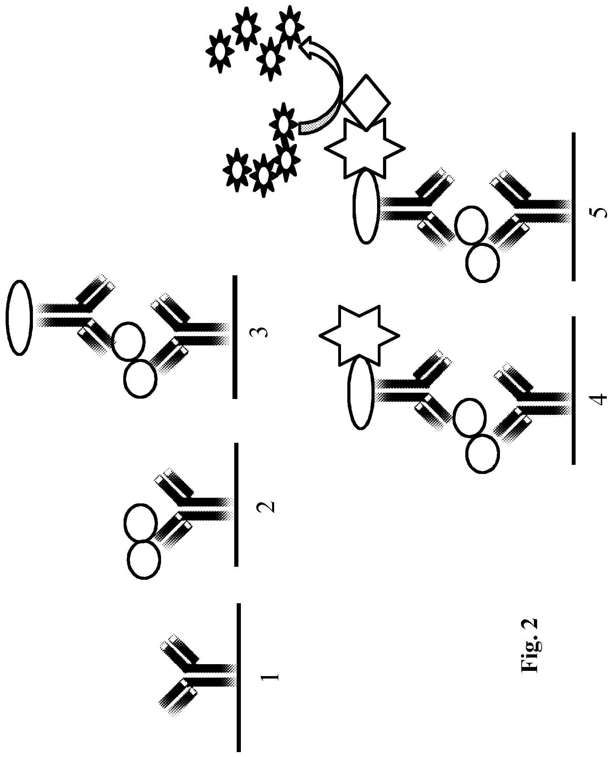

In an embodiment, the kit also comprises a microtiter plate (MCP) as the support to which the first antibody is immobilized or is intended to be immobilized. Fig. 2 is a schematic overview of the concept of a sandwich ELISA using the first and second antibodies according to an embodiment.

The kit does not necessarily have to be an ELISA kit. In another embodiment, the kit uses affinity chromatography where the first antibody is bound to the stationary phase, such as to a gel matrix or beads in a column. For instance, the gel matrix or beads could be made of agarose, such as SEPHAROSE^{®}.

In such a case, non-human mammal TK1 protein present in a sample will be entrapped in the column through binding to the immobilized first antibodies. Following washing, the bound non-human mammal TK1 protein can be eluted and detected using the second antibody. For instance, the amount of eluted non-human mammal TK1 protein can be determined using Western blotting and with the second antibody for TK1 detection using direct or indirect detection methods.

The support could alternatively be magnetic beads, such as DYNABEADS^{®} magnetic beads.

The non-human mammal TK1 protein determined can be non-human mammal cellular and/or serum TK1 protein, preferably non-human mammal serum TK1 (STK1) protein or molecules.

According to the invention, the non-human mammal TK1 protein is selected from a group consisting of canine TK1 protein, feline TK1 protein and equine TK1 protein. Hence, according to the invention, the TK1 protein can be from dogs, cats or horses. In a particular embodiment, the non-human mammal TK1 protein is canine TK1 protein.

The kit of the present embodiments can be used in an *in vitro* method for determining the level of non-human mammal TK1 protein in a sample.

Another aspect relates to an *in vitro* method for determining a level of canine, feline or equine TK1 protein in a sample. The method comprises contacting the sample with a first antibody and a second antibody of the kit according to the embodiments. The method also comprises detecting an amount of bound second antibody.

The level of canine, feline or equine TK1 protein in the sample is then determined based on the detected amount of bound second antibody.

Any prior art techniques to detect the amount of bound antibody can be used in the present method. For instance, the detection can be direct or indicted, and may generate a fluorescent or chromogenic signal. Direct detection typically involves the use of an antibody that is conjugated to a label. Indirect detection utilizes a labeled secondary antibody raised against the host species of the antibody.

Commonly used labels for visualization of binding of antibody to epitope includes fluorophores and enzymes that convert soluble substrates into chromogenic end products.

The sample is a body sample and is more preferably selected from a group consisting of a cell sample, a tissue sample, a blood sample, a serum sample, a cerebrospinal fluid sample, a pleural fluid sample, a synovial fluid sample and a peritoneal cavity fluid sample. In a preferred embodiment, the body sample is a body fluid sample and preferably selected from a group consisting of a blood sample, a serum sample, a cerebrospinal fluid sample, a pleural fluid sample, a synovial fluid sample and a peritoneal cavity fluid sample. In a particular embodiment, the body (fluid) sample is a blood sample or a serum sample.

Thus, the antibodies can be used to determine a level, i.e., an amount, of non-human mammal TK1 protein in a sample. The antibodies are believed to be able to bind to and thereby enable determination of the level of non-human mammal cellular or serum TK1 in its various forms, such as dimers, tetramers, and oligomers, including potentially bound to further proteins, cofactors or molecules. Thus, non-human mammal cellular and/or serum TK1 protein thereby includes the non-human mammal cellular and/or serum TK1 in its various forms.

The method may be a method for determining a level of non-human mammal STK1 protein in a body sample.

The method may be a method for determining a level of non-human mammal cellular TK1 protein in a body sample.

The method may be a method for determining a level of non-human mammal cellular TK1 protein and non-human mammal STK1 protein in a body sample.

A further aspect of the embodiments relates to an *in vitro* method for estimating the likelihood of recurrence of a tumor disease in a canine, feline or equine subject, i.e., a dog, a cat or a horse according to the claims. The method comprises determining a level of canine, feline or equine TK1 protein in a body sample from the canine, feline or equine subject using the *in vitro* a method or the kit according to the embodiments. The level of canine, feline or equine TK1 protein in the body sample is then compared with a level of canine, feline or equine TK1 protein representative of a population of healthy canine, feline or equine subjects or with a level of canine, feline or equine TK1 protein previously determined in the canine, feline or equine subject. The method further comprises estimating the likelihood of recurrence of the tumor disease in the non-human mammal subject based on the comparison.

A determined level that is higher than a level associated with a population of healthy non-human mammals indicates an increased likelihood of recurrence of a tumor disease in the non-human mammal subject. Similarly, a determined level that is higher than a level associated with the non-human mammal subject subsequent to previous therapy indicates an increased likelihood of recurrence of a tumor disease in the non-human mammal subject.

Yet another aspect of the embodiments relates to a method for determining cell proliferation in a canine, feline or equine subject according to the claims. The method comprises determining a level of canine, feline or equine TK1 protein in a body sample from the canine, feline or equine subject using the *in vitro* a method or the kit according to the embodiments. The method also comprises determining the cell proliferation based on the level of canine, feline or equine TK1 protein in the body sample.

A level of normal or tumor cell proliferation is determined and compared with the determined level of non-human mammal TK1 protein to determine whether the non-human mammal subject has normal or baseline cell proliferation or an elevated cell proliferation.

The present method can be used as a tool in monitoring various therapies applied to non-human mammal subjects. For instance, the method can be used to monitor anti-proliferation or anti-tumor therapy in the non-human mammal subject. In such a case, the method can be used to verify whether a selected anti-proliferation or anti-tumor therapy has the desired effect in reducing cell proliferation in the non-human mammal subject. If the therapy does not have the desired effect, i.e., no significant decrease in cell proliferation is detected, then another or a modified anti-proliferation or anti-tumor therapy can be applied to the non-human mammal subject.

A further aspect of the embodiments relates to a method for determining a proliferation process response in a canine, feline or equine subject suffering from a malignant disease according to the claims. The method comprises determining a level of canine, feline or equine TK1 protein in a body sample from the canine, feline or equine subject using the *in vitro* a method or the kit according to the embodiments. The method also comprises determining the proliferation process response based on the level of canine, feline or equine TK1 protein in the body sample. An example of such a proliferation process response could be an immune reaction or immune reaction response.

At least one other biomarker for the proliferation process response may also be usec in the determination.

Yet another aspect of the embodiments relates to a method for determining a level of inflammation, infection or tumor cell proliferation in a canine, feline or equine subject according to the claims. The method comprises determining a level of canine, feline or equine TK1 protein in a body sample from the canine, feline or equine subject using the *in vitro* a method or the kit according to the embodiments. The method also comprises determining the level of inflammation, infection or tumor cell proliferation based on the level of canine, feline or equine TK1 protein in the body sample. In an embodiment, at least one other biomarker for inflammation, infector or tumor cell proliferation may also be used in the determination.

A further aspect of the embodiments relates to a method for evaluating efficiency of a treatment of a malignant disease in a canine, feline or equine subject according to the claims. The method comprises determining a level of canine, feline or equine TK1 protein in a body sample from the canine, feline or equine subject using the *in vitro* a method or the kit according to the embodiments prior to or in connection with start of the treatment of the malignant disease. The method also comprises determining a level of canine, feline or equine TK1 protein in a body sample from the canine, feline or equine subject using the method or kit according to the embodiments during or after the treatment of the malignant disease. The method further comprises evaluating efficiency of the treatment of the malignant disease based on a comparison of the level of canine, feline or equine TK1 protein determined in the body sample prior to or in connection with start of the treatment of the malignant disease and the level of canine, feline or equine TK1 protein determined in the body sample during or after the treatment of the malignant disease.

The *in vitro* method and kit can be used to evaluate or determine efficiency of a treatment of a malignant disease in dogs, cats or horses. Thus, by comparing determined TK1 protein levels during or after the treatment with corresponding TK1 protein levels previously determined before the start of the treatment or in connection with the start of the treatment it is possible to determine whether the selected treatment has any medical effect in terms of reducing the TK1 protein level in the non-human mammal. Hence, a reduction in TK1 protein level in a treated non-human mammal is determined to correlate with a treatment that has effect with regard to the malignant disease, i.e., an efficient treatment. However, if no significant reduction in TK1 protein level is detected in a treated non-human mammal, then the particular treatment is not efficient and does not have the desired effect with regard to the malignant disease.

The tumor is preferably a hematological tumor, such as lymphoma or leukemia, or a solid tumor, such as mammary tumor, histiocytic sarcoma, mastocytoma, melanoma, hemangiosarcoma or adenocarcinoma. Kiran Kumar 2010 disclosed production of anti-dog TK1 antibodies produced by immunizing rabbits with a 28 amino acid long peptide corresponding to amino acids 196 to 223 in dog TK1. The anti-dog TK1 antibodies, however, showed poor performance when used in an ELISA assay and had high background. Production of polyclonal antibodies against the long (28 amino acids) peptide showed large batch-to-batch variation. This is in clear contrast to present embodiments and the experimental data presented herein. The antibodies of the present embodiments can successfully be used in an ELISA assay with high specificity and capable of discriminating between healthy subject and subjects suffering from hematological or solid tumors.

### EXAMPLES

### EXAMPLE 1

A new TK1-ELISA for determination of serum TK1 (STK1) protein levels in dogs with hematological and solid tumors was developed. Both STK1 activity and STK1 protein levels were measured in sera from healthy dogs, dogs with leukemia and lymphomas, and solid tumors. Furthermore, serum samples from six dogs with lymphoma were followed during therapy to test the monitoring efficiency of both these TK1 assays.

### Materials and Methods

### Serum samples

Serum samples from healthy dogs, dogs with hematological tumors and solid tumors were collected from the University Animal Hospital at the Swedish University of Agricultural Sciences, Uppsala, Sweden, and stored at -20°C until analysis. The project was approved by the Swedish Animal Ethics Committee. The study comprised samples from healthy dogs (n = 30), dogs with hematological tumors (n = 36: lymphoma, n = 31, leukemia n = 5) and dogs with solid tumors (n = 40). The mean and median age was 6 years (range 3-10 years) for the healthy group, 8.5 and 8 years (range 3-13 years) for hematological tumors, and 9 and 8 years (range 3-14 years) for dogs with solid tumors.

The tumor diagnostic procedures were as described previously (von Euler et al., 2008). A total of 35 breeds were included in this study. The most common breeds included were Labrador Retriever (13/105), Golden Retriever (12/105), Rottweiler (11/105), Mixed breeds (11/105), Riesen Schnauzer (11/105), German Shepherd (7/106), Flat coated Retriever (5/105), Nova Scotia Retriever and Boxer (3/105 of each) followed by Norwich Terrier, Fox Terrier, Bull Terrier, Bernese Mountain dog, Rhodesian ridge back, English Springer Spaniel and Mixed breeds (2/105 of each), Clumber Spaniel, Scottish Terrier, Doberman, Hovawart, Norwich Terrier, West highland Terrier, Shetland Sheep Dog, Swedish Elk Hound, Field Spaniel, Leon Berger, Brooder Coolie, Grey Hound, Cocker Spaniel, French Bull dog, Weimaraner, Pug and Australian Shepherd (1/105).

### TK1 antibodies

Polyclonal antibodies were produced against a peptide in the C-terminal region of dog TK1. The 16-mer antibody was produced using a 16 amino acid synthetic peptide (amino acids 215-230, GKPGEGKEATGVRKLF, SEQ ID NO: 1; PAb-Arv1) (Fig. 1) with a cysteine added to the N-terminus. This 16-mer peptide was coupled to keyhole limpet hemocyanin (KLH) and mixed with Freund's complete adjuvant as antigen for immunization of rabbits (GenScript, Piscataway, NJ, USA). The antisera were collected after the 3^{rd} and 4^{th} immunizations and purified on peptide coupled Sepharose 4B columns as previously described (Wu et al., 2003). The 24-mer antibody was produced as previously described (Gasparri et al., 2009) against the long lasso shaped loop in the active site of human TK1 (amino acids 161-183, AYTKRLGTEKEVEVIGGADKYHS, SEQ ID NO: 2; Ar-4) with a cysteine added to the N-terminus. A 24-mer peptide was also coupled to KLH and mice were immunized and monoclonal antibodies were prepared as previously described (Gasparri et al., 2009). The Ar-4 antibodies were provided by AroCell AB, Uppsala, Sweden.

### Biotinylation of Ar-4

The Ar-4 antibodies were biotinylated using the ChromaLink^{™} Biotin Antibody Labeling Kit (Solulink, California, USA) according to manufacturer instructions. The biotinylated Ar-4 antibodies were analyzed by Western blotting using streptavidin-HRP.

### The serum TK1 activity assay

TK1 activity in serum samples was determined using the optimized [³H]-dThd phosphorylation assay, as described previously (Sharif et al., 2012 a; Kiran Kumar et al., 2013). In brief, 10 µL of serum was incubated with the reaction mixture containing 10 mM Tris-HCl pH 7.6, 2 mM dithiothreitol (DTT), 5 mM MgCl₂, 5 mM NaF, 5 mM ATP, and 5 µM [³H]-deoxy thymidine for 1 h at 37°C and applied to the DE-81 filter paper discs. The filters were then washed twice with 1 mM ammonium formate for 5 min/each and the bound products were eluted for 45 min in 0.1 M HCl and 0.2 M KCI. The radioactivity was determined by β-scintillation counting as previously described (Sharif et al., 2012 a). The TK1 activity was expressed as pmol/min/mL.

### Development of a sandwich ELISA to detect TK1 in dog sera

ELISA plates of the type Maxisorp, NUNC (Denmark) were coated with 100 µL of the PAb-Arv1 anti-TK1 polyclonal antibody (4 µg/mL) in carbonate buffer (pH 9.6) and incubated overnight at 4°C. Plates were washed, using a Tecan Hydro flex microplate washer, four times with wash buffer (Tris 0.1 M, pH 7.4, NaCl 0.3 M, Tween 0.05 % and BSA 1 %). Thereafter, all wells were blocked with 5 % nonfat dry milk powder diluted in TBST for 1 h at room temperature. Dog serum samples of 50 µL were diluted in 50 µL serum dilution buffer (AroCell AB, Uppsala) and pre-incubated at room temperature for 1 h. Wells were again washed and pre-incubated serum samples were added. Then plates were incubated at room temperature for 2 h on a rocking platform. Plates were washed as described above and the biotinlayted Ar-4 antibody (4 µg/mL in wash buffer) was added. After incubation at room temperature for 1 h, the plates were washed as above and horse radish peroxidase (HRP) diluted 1:32,000 in wash buffer was added, and incubated for 1 h. After the final wash, TMB (Thermo Fisher Scientific) was added and allowed to develop for 15 min, and then quenched with 2N H₂SO₄. The plates were read at 450 nM in an Infinite M200 Micro plate reader (Tecan, Mannedbrf, Germany).

### Validation of the dog TK1 ELISA

Dog recombinant TK1 was purified as described previously (Sharif et al., 2012 b) and a standard curve was established with different concentrations of recombinant dog TK1 (0.6 - 10 ng /ml diluted in sample dilution buffer). By using the standard curve, based on average absorbance of different serum samples, the concentrations of TK1 in the serum samples were calculated. The detection limit was estimated as the minimum analyte concentration giving a value significantly different from that of the zero calibrator. The inter-assay variation was also determined from the mean and SD of all serum samples which were independently assayed as duplicates in two different experiments.

### Statistical analysis

The TK1 activity and TK1 protein distribution in healthy, hematological tumors and solid tumors were tested for normality using the D'Agostino and Pearson omnibus normality test. STK1 activity and protein levels in healthy dogs as well as dogs with hematological and solid tumors showed non-Gaussian distribution. Spermann correlation coefficient (rs) was used to determine the correlation between TK1 activities and TK1 protein levels. Mann Whitney t-test was used to evaluate the difference between the groups. For sensitivity and specificity analysis and for comparison of assays, receiver operating characteristic (ROC) curves were constructed. Statistical analyses were performed using Graph Pad Prism 5.0 (Graph Pad Software, La Jolla, CA, USA). The level of significance was set at P<0.05.

### Results

### Description of the ELISA procedure

The ELISA principle is based on a sandwich immunoenzymatic system as shown in Fig. 2. The first step is a coating of micro titer plates with purified polyclonal anti dog TK1 specific antibody produced against a peptide in the C-terminal region of dog TK1 (PAb-Arv1, Fig. 1). This part of TK1 is an exposed region of the TK1 protein complexes formed in the blood. After blocking the wells with milk powder (5 %), pre-incubated serum samples from healthy and tumor dogs were allowed to bind to the antibodies on the plates. Proteins not specifically bound are removed by the washing procedure. The second antibody Ar-4 made against a highly conserved and exposed active site region of TK1 were biotinylated and allowed to react with the TK1 bound to the wells. The detection of antigen-antibody bound complex by a streptavidin-peroxidase (HRP) complex, which is visualized by the addition of a chromogenic substrate (TMB). The intensity of the color reaction is proportional to the quantity of TK1 present in the serum samples.

### Detection limit and precision of ELISA

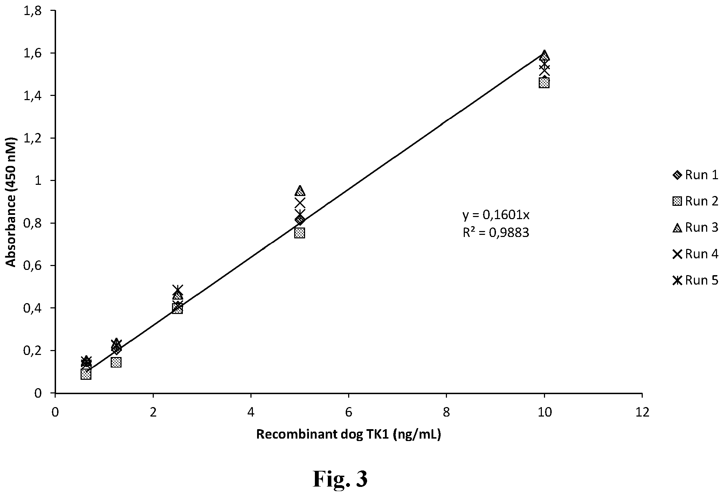

Dog recombinant TK1 was used as a calibrator to generate the dose-response curve of the TK1- ELISA. A typical calibration curve from 5 different runs is shown in Fig. 3. Intra assay variation at all non-zero calibration points CVs were ≤ 10 %. The limit of detection (LOD) of the assay was 0.46 ng/mL and between-run imprecision (CV) was <20 % at concentrations down to 0.63 ng/mL. In case of serum samples the inter-assay variation ranged from 5-15 % and intra assay variation was 5 %. The median serum TK1 activities and TK1 protein levels from the healthy dogs, dogs with hematological malignancies and dogs with solid tumors are summarized in Table 1 below.

**Table 1 - TK1 activity and protein levels in sera from healthy dogs, dogs with hematological malignancies and dogs with solid tumors**

| | **No of samples** | **STK1 activity (pmol/min/mL)** | | **STK1-ELISA (ng/mL)** | |
|---|---|---|---|---|---|
| **Group** | **(N)** | **Range** | **Median** | **Range** | **Median** |
| Healthy | 30 | 0.7 - 1.4 | 1.02 | < 0.46 - 0.47 | < 0.46 |
| Lymphoma | 31 | 0.54 -29.8 | 2.43 | < 0.46 - 4.38 | 0.78 |
| Leukemia | 5 | 1.33 - 58.7 | 38.3 | < 0.46 - 4.19 | 3.92 |
| Mammary tumors | 16 | 0.62 - 1.83 | 1.07 | < 0.46 -1.51 | 0.50 |
| Mastocytoma | 8 | 0.51 - 1.52 | 0.85 | < 0.46 - 1.1 | 0.50 |
| Malignant melanoma | 11 | 0.52 - 1.52 | 1.15 | < 0.46 - 3.40 | 0.53 |
| Other tumors | 5 | 0.53 - 1.78 | 1.08 | < 0.46 -1.13 | 0.67 |

| | | | | | |
|---|---|---|---|---|---|
| TK1-ELISA values below LOD are represented as less than 0.46 ng/mL | | | | | |

### STK1 activity levels in healthy dogs and dogs with hematological tumors

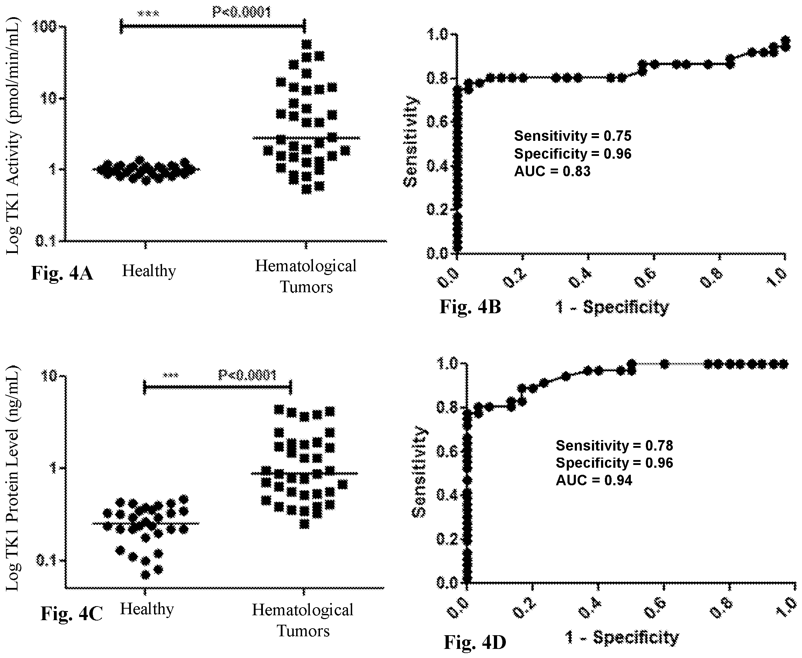

In the healthy dog group, STK1 activities were in the range of 0.7 - 1.4 pmol/min/mL (Table 1) with a median of 1.02 pmol/min/mL. The STK1 activity levels in sera from dogs with hematological tumors ranged from 0.5 - 59 pmol/min/mL (Table 1). In healthy dogs as well as in dogs with hematological tumors, no significant difference was found in STK1 activity levels between males and females. Statistical analysis using the Mann-Whitney U test showed that STK1 activity was significantly higher in the hematologic tumors group compared to in healthy dogs (P<0.0001, Fig. 4A). The ROC curve analysis of STK1 activity assay results showed an area under curve (AUC) of 0.83 (P< 0.0001, 95 % Cl, 0.72-0.94) (Fig. 4B). At the cut-off value of 1.35 pmol/ min/mL, the sensitivity was 75 % (95 % Cl, 0.578-0.878) and the specificity 96 % (95 % Cl, 0.82-0.99).

### TK1 ELISA determinations in healthy dogs and dogs with hematological malignancies

TK1 protein levels in clinical samples were determined by using different concentrations of recombinant dog TK1 as standard. In healthy dogs, STK1 protein levels were < 0.46 ng/mL and 1 out of 30 sera had protein value of 0.47 ng/mL. The estimated upper limit of the normal reference based on the 30 healthy dogs was 0.46 ng/mL. Sera from dogs with lymphoma or leukemia had in general higher STK1 protein levels, ranging from < 0.46 to 4.4 ng/mL. Significant differences were found in the median STK1 protein levels between healthy and hematological tumors (P<0.0001, Fig. 4C). The ROC curve analysis showed an AUC of 0.94 (P< 0.0001, 95 % Cl, 0.89-0.99). The true positive rate was 78 % (95 % Cl, 0.60-0.898) and the false positive rate was 4 % (95 % Cl, 0.82-0.99), using a cutoff value of 0.46 ng/mL (Fig. 4D).

### STK1 activity and protein levels in sera from dogs with solid tumors

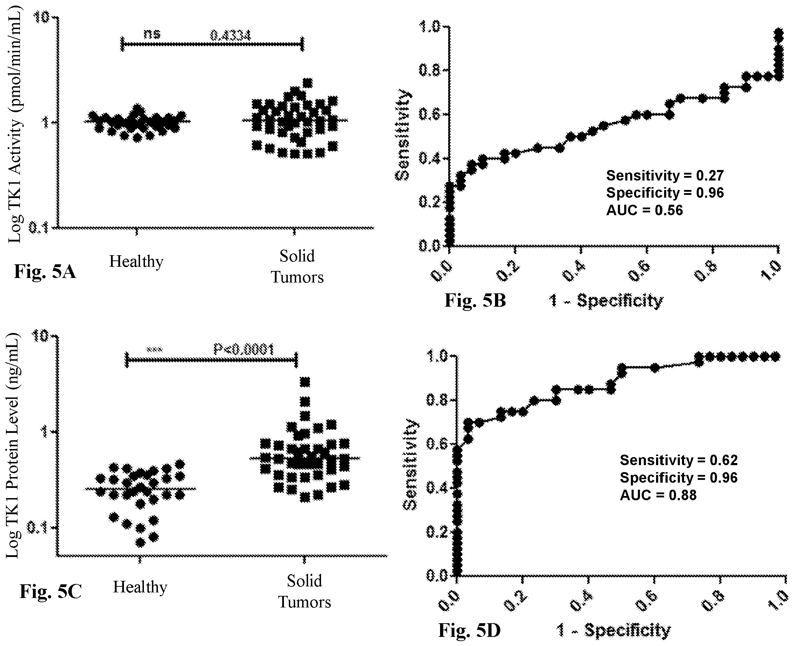

Most of the serum samples from dogs with solid tumors had STK1 activity below the cut-off value. STK1 activity levels ranged from 0.5 - 2.5 pmol/min/mL (the median value = 1.02). There was no significant difference (P = 0.193) between the solid tumors and the healthy groups (Fig. 5A). The ROC curve analysis showed an AUC of 0.56 (P = 0.43, 95 % Cl, 0.41-0.69). The true positive rate was 27 % (95 % Cl, 0.14-0.43) and the false positive rate was 4 % (95 % Cl, 0.82-0.99), using a cutoff value of 1.35 pmol/min/mL (Fig. 5B). However, the TK1 protein levels were significantly higher in sera from dogs with solid tumors (range of < 0.46 to 3.4 ng/mL) compared to healthy (P<0.0001, Fig. 5C). Furthermore ROC curve analysis had an AUC of 0.88 (P< 0.0001, 95 % Cl, 0.80-0.95) (Fig. 5D). At the cut-off value of 0.46 ng/mL, the sensitivity was 62 % (95 % Cl, 0.458-0.772) and the specificity 96 % (95 % Cl, 0.82-0.99). These results strongly indicate that the TK1-ELISA could differentiate dogs with solid tumors from healthy dogs.

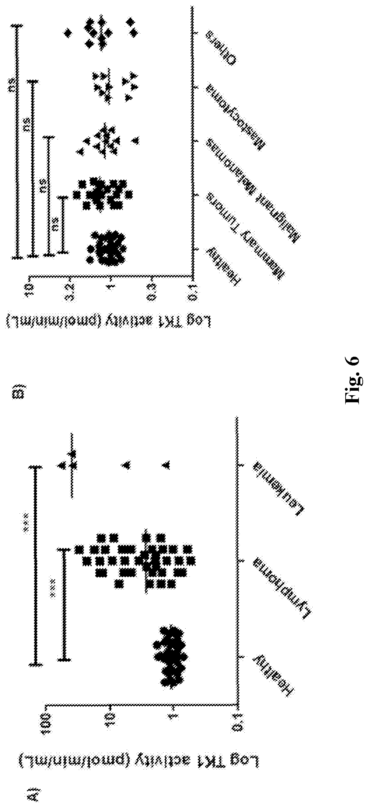

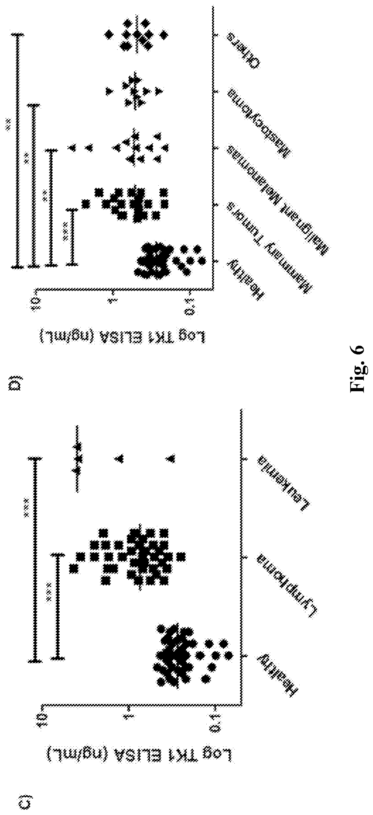

Over all, STK1 activity assay values in healthy, hematological and solid tumor dogs showed significant correlation to the STK1 ELISA (ng/mL) values, i.e., with r = 0.64 (P<0.0001) using linear regression analysis. Sera from dogs with hematological and solid tumors were further sub-classified as lymphomas, leukemias and mammary tumors, mastocytomas and melanomas. Figs. 6A and 6B illustrate the log STK1 activity distribution in the different sub-classes, whereas Figs. 6C and 6D illustrate the log STK1 protein levels in the different sub-classes.

### Serum TK1 activity and protein levels followed during chemotherapy of dogs with lymphoma

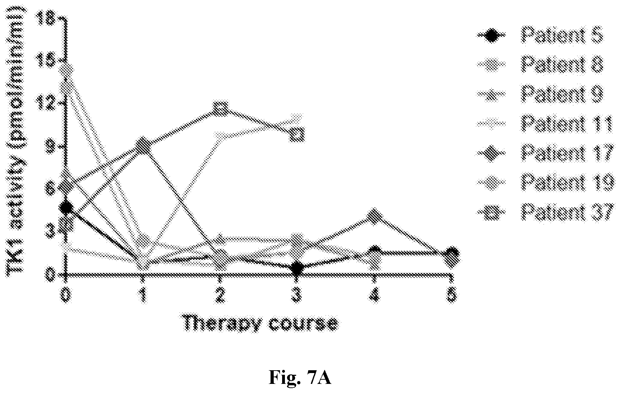

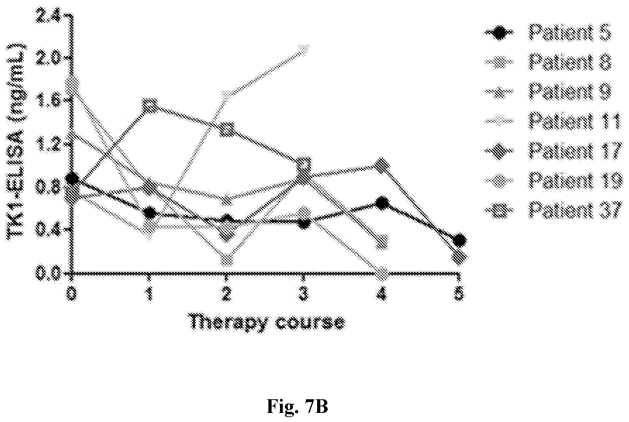

Dogs with lymphoma are often treated using a doxorubicin based multi-agent protocol (ADRIA-Plus). Serum samples were collected from six dogs before and after each dose of ADRIA-plus. Initially, high STK1 activity and STK1 protein values were found in five dogs and four out of the six dogs showed significant decline in STK1 activity and STK1 protein levels after the first treatment, leading to levels similar or lower than the cut-off value (Figs. 7A, 7B). In two patients (no. 8, 9) there was an increase in STK1 activity and STK1 protein during 3^{rd} treatment but the levels decreased after the 4^{th} treatment. In two patients (patient no. 5, 17) STK1 activity and STK1 protein levels increased after the 1^{st} and decreased after 2^{nd} treatment but increased again after the 3^{rd} and 4^{th} treatment followed by a decline after the 5^{th} treatment. One patient (patient no. 19) was apparently in complete remission after the 2^{nd} treatment. Mean STK1 activity and STK1 protein in dogs with lymphoma that were in complete remission were not significantly different from the STK1 in healthy controls. However, two patients (patient no. 11, 37) showed a different pattern of response to chemotherapy. Patient no. 11 had significant reduction in STK1 activity and STK1 protein levels after 1^{st} treatment. Thereafter, STK1 levels were increased after 2^{nd} and 3^{rd} treatment and this patient had tumor relapse, which did not respond further to the treatment. Patient no. 37 had significant increase in STK1 activity and STK1 protein levels after 1^{st} treatment. During the further course of therapy, a slight decrease was found in STK1 levels after 2^{nd} and 3^{rd} treatments, still both the STK1 activity and STK1 protein levels were significantly higher compared to controls. Furthermore, this dog also did not respond to the treatment and both the dogs were euthanized later. The results shown here strongly indicate that both STK1 activity and STK1 protein assays may provide information concerning the prognosis and efficiency of treatment of canine lymphomas.

### Discussion

Previous studies have demonstrated that serum TK1 activity measurements are valuable for prognosis and treatment monitoring of canine hematological tumors. However, these activity assays are complex as they involve use of hazardous and expensive radioisotopes, thus limiting the clinical utility of the TK1 activity assays. Since ELISA is commonly used in almost all clinical laboratories, developing ELISA immunoassays would significantly increase the clinical utility of TK1 as biomarker. In human medicine studies with immunoassays based on TK1 antibodies showed that they were sensitive tools for prognosis and monitoring of different tumor diseases. There have been several attempts to establish antibody based TK1-ELISA in human medicine but only very recently a robust ELISA is available as a research use only product (AroCell AB, Sweden). This has probably been due to the difficulty to obtain antibodies with sufficient affinity for the serum forms of TK1. The complexity of TK1 oligomers found in serum, most likely prevent reproducible reactivity with different type of anti TK1 antibodies.

In one preliminary study antibodies directed against the long lasso loop (amino acids 161-183) in the active site of human TK1 was used. This region is highly conserved in the dog TK1 sequence (Fig. 1) and was also used for production of Ar-4 (von Euler & Eriksson, 2011). It was clear that these antibodies detected cellular TK1 in canine lymphoma tissues. In another study, using two antibodies raised against peptides from the C-terminal region of dog TK1 recombinant, cellular and serum forms of the canine TK1 could be detected and characterized (Sharif et al., 2012 b). There are several similarities but also differences particularly in the oligomeric structure of human and the dog serum TK1.

A recent study reported that an immunoaffinity assay with dog anti-TK1 antibodies can measure TK1 levels in sera from hematological and solid tumors, and indicate that TK1 protein assays were more sensitivity than TK1 activity assays (Kiran Kumar et al., 2013). Furthermore, studies have shown that sera from dogs with solid tumor had low TK1 activity but relatively high levels of TK1 protein compared to healthy dogs.

A canine TK1-ELISA was developed as disclosed herein, which can overcome the limitations of traditional TK1 radioisotope assays. ROC curve analysis for hematological tumors demonstrated that both the TK1-ELISA and TK1 activity assays have a sensitivity of 75 % and a false positive rate of 4 %. This indicates that both assays may be accurate and sensitive enough for clinical routine practice. However, TK1 activity assay could not distinguish healthy from solid tumor patients, which were demonstrated by the ROC curve with a much lower sensitivity (27 %). The TK1-ELISA could differentiate these groups with two fold higher sensitivity (62 %) than the TK1 activity assay. Furthermore, TK1 protein levels return to normal (baseline level) after chemotherapy leading to tumor remission and increased to higher levels in relapsed patients, similar to TK1 activity levels in dogs with lymphoma. A transient increase in TK1 protein levels were found in a few patients during therapy, which could be due to release of TK1 from cells that died because of drug toxicity.

In addition, size exclusion analysis from previous studies showed that serum TK1 exist as high molecular weight aggregates, which is enzymatically active in case of dog lymphomas. Interestingly, sera from mammary tumor dogs had a small portion of TK1 high molecular weight aggregates that were active, but a large fraction of the protein was apparently inactive (Jagarlamudi et al., 2014). Still a strong correlation between results with the two assays (rs = 0.64) was found. This indicates that the molecular forms of TK1 contribute to the overall correlation observed between the two assays. The availability of a new ELISA will increase the clinical utility of TK1 as a proliferation biomarker and should be valuable for prognosis and monitoring hematological tumors in veterinary medicine.

The present study describes a new TK1-ELISA for determining TK1 protein levels, which can be a potential marker for diagnosis and monitoring of canine hematological tumors. The TK1-ELISA is easy to perform, fast and as sensitive and specific as the existing TK1 activity assays.

### EXAMPLE 2

The following experiments demonstrated the reactivity of dog TK1 antisera raised against peptides from different regions of the dog and catTK1 sequences. Most of the peptide sequences of TK1 are conserved in human, dog, cat and horse TK1 sequences except a few amino acid differences, see Fig. 1. However, there are considerably more divergences between the TK1 sequences in the C-terminal end of the TK1 proteins.

### TK1 antisera

Two different polyclonal rabbit antisera were produced using peptides from the C-terminal region of dog TK1 (CTK1p-211, CTK1p-215) and one antisera produced from the active site region of dog TK1 (CTK1p-161). The first canine TK1 peptide sequence (CTK1p-215) antiserum was against a 16 amino acid synthetic peptide (amino acids 215-230 in dog TK1; Fig. 1) to which a N-terminal cysteine was added (CGKPGEGKEATGVRKLF, SEQ ID NO: 5). The second antiserum (CTK1p-211) was produced using a 15 amino acid peptide (amino acids 211-225 in dog TK1; Fig. 1) to which a N-terminal cysteine was added (CVLVPGKPGEGKEATG, SEQ ID NO: 12). The CTK1p-161 antisera was produced using a highly conserved active site sequence of dog TK1 (amino acids 161-183 in dog TK1; Fig. 1) to which a N-terminal cysteine was added (CAYTKRLGTEKEVEVIGGADKYHS, SEQ ID NO: 8). FTK1p-213 antisera was produced against the C-terminal region of cat TK1 (amino acids 213-227 in cat TK1; Fig. 1) to which a N-terminal cysteine was added (CGKPGEASGARKLFAP, SEQ ID NO: 14). All these peptides had a cysteine added to the N-terminus for coupling to the carrier protein KLH. The peptide carrier complexes were used as antigens for immunization of the rabbits (GenScript, Piscataway, NJ, USA). The tested antisera were collected after the 3^{rd} and 4^{th} immunizations. Cloning and expression of the cat and the horse recombinant TK1 protein was done as described for the dog TK1 protein (Jagarlamudi et al., 2015).

### Dot blot immunoassay

All four different TK1 antisera were tested for their reactivity with recombinant dog, cat and horse TK1 by using a dot blot assay. In brief, 3 µL of different dilutions of the recombinant TK1 preparations were applied on nitrocellulose membrane (Bio-Rad). Membranes were allowed to dry and blocked with 5 % non-fat dry milk powder diluted in TBST (mixture of tris-buffered saline (TBS) and Polysorbate 20 (also known as Tween 20)) for 1 hour at room temperature. The membranes were washed three times with TBST and then incubated with the different TK1 antisera diluted in TSBT (1:300) for 2 hours at room temperature. The washing procedure was repeated and the membranes were incubated with streptavidin horse radish peroxidase (HRP) (dilution: 1:20,000 in TBS) for 1 hour. After a final washing, the membranes were incubated for 1 min with ECL chemiluminiscence (GE health care, Uppsala). The membranes were scanned with Bio-Rad chemi doc touch system. The intensities of dots were quantified with image gauge 3 software.

### Results and Discussion

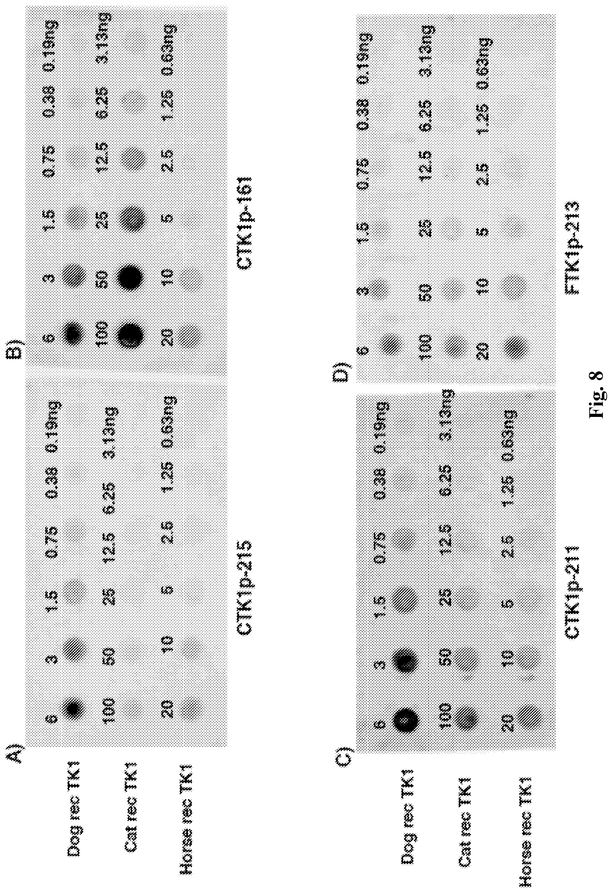

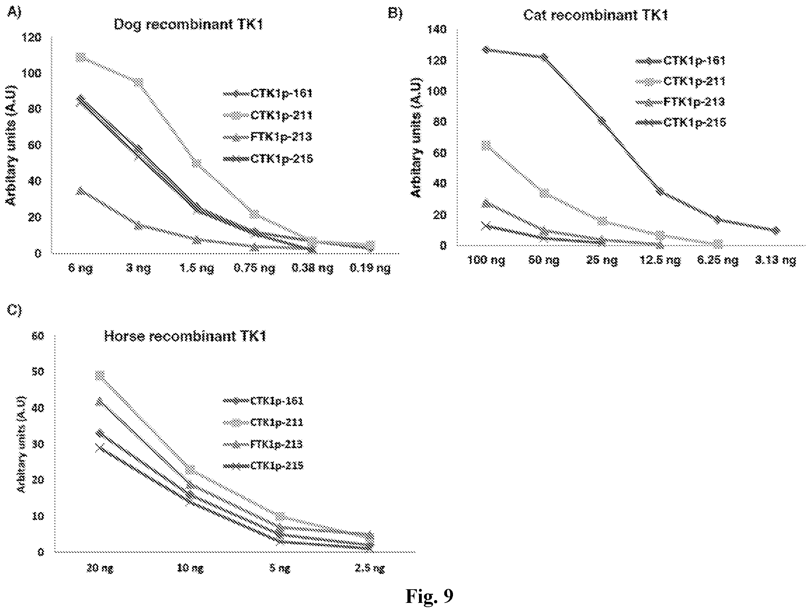

Figs. 8A-8D illustrate the results of the dot blot immunoassay with dog, cat and horse recombinant TK1 with the four different TK1 antisera. Figs. 9A-9C illustrate dot intensities in arbitrary units with different concentrations of dog, cat and horse recombinant TK1 with the four different TK1 antisera.

As is shown in Figs. 8 and 9, there are large differences in the capacity of various C-terminal peptides (CTK1p-215, CTK1p-211 and FTK1p-213) to induce production of antibodies that react with recombinant dog, cat and horse TK1. Apparently, CTK1p-211 gave antibodies with high reactivity with all tested recombinant TK1 proteins, but with a clear preference for dog TK1. CTK1p-215 gave antisera against dog and horse TK1, while FTK1p-213 only produced antibodies reactive with horse TK1. CTK1p-161 resulted in reacting antibodies with all three types of TK1 protein and was superior for cat TK1.

### EXAMPLE 3

The procedure in Example 1 was repeated but with the following differences.

### Sandwich ELISA procedure to detect TK1 in dog sera

Maxisorp, NUNC (Denmark) micotitre plates were coated with 100 µL of the CTK1p-215 anti-TK1 rabbit polyclonal antibody (4 µg/mL) in carbonate buffer (pH 9.6) and incubated overnight at 4°C. Plates were washed, using a Tecan Hydro flex microplate washer, four times with wash buffer (Tris 0.1 M, pH 7.4, NaCl 0.3 M, Tween 0.05 % and BSA 1 %). Thereafter, all wells were blocked with 5 % nonfat dry milk powder diluted in TBST for 1 h at room temperature. Dog serum samples of 50 µL were diluted in 50 µL sample dilution buffer and pre-incubated at room temperature for 1 h. Plates were again washed and pre-incubated serum samples were added. Then, plates were incubated at room temperature for 2 h on a rocking platform. Plates were washed as described above and the biotinylated CTK1p-161 antibody (4 µg/mL in wash buffer) was added. After incubation at room temperature for 1 h, the plates were washed as above and horse radish peroxidase (HRP) (Thermo Fisher Scientific) diluted 1:10,000 in wash buffer was added, and incubated for 30 min. After the final wash, TMB (Thermo Fisher Scientific) was added and allowed to develop for 15 min, and then quenched with 2N H₂SO₄. The plates were read at 450 nM in an Infinite M200 Micro plate reader (Tecan, Mannedbrf, Germany) using 540 nM as a reference wavelength.

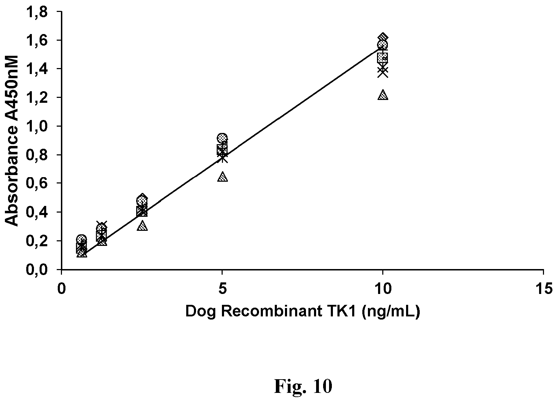

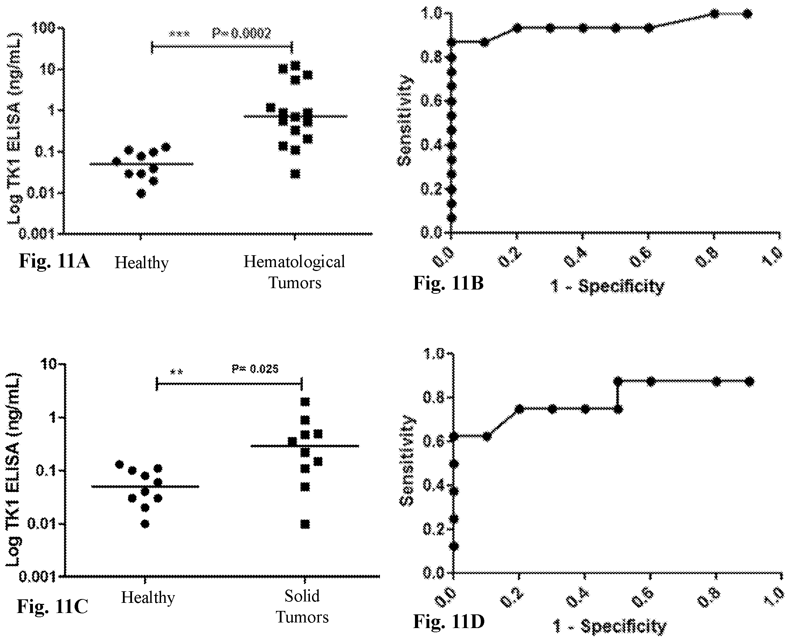

The results from this Example 3 are presented in Figs. 10 and 11A-11D. In more detail, Fig. 10 illustrates a standard curve with different concentrations of recombinant TK1 with CTK1p-215 coating and CTK1p-161 as detection antibodies. Fig. 11A illustrates the log STK1 ELISA protein levels in sera from healthy dogs and dogs with hematological malignancies. Figs. 11B illustrates the corresponding ROC curve for discrimination of hematological tumor dogs from healthy dogs. Fig. 11C illustrates the log STK1 ELISA protein levels in sera from healthy dogs and dogs with solid tumors. Figs. 11D illustrates the corresponding ROC curve for discrimination of solid tumor dogs from healthy dogs.

The same procedure was used but antibodies were changed. CTK1p-211 anti-TK1 polyclonal antibody was used for coating and biotinylated CTK1p-215 was used for detection.

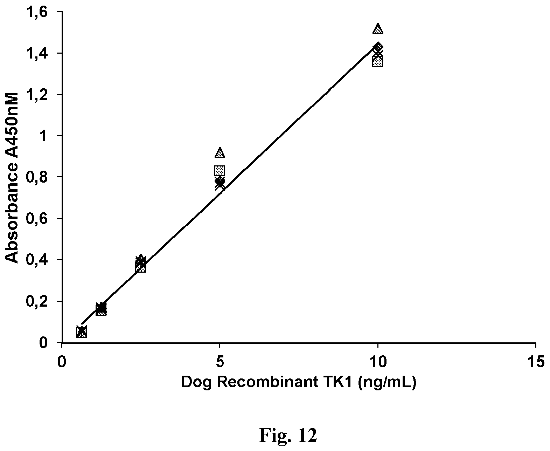

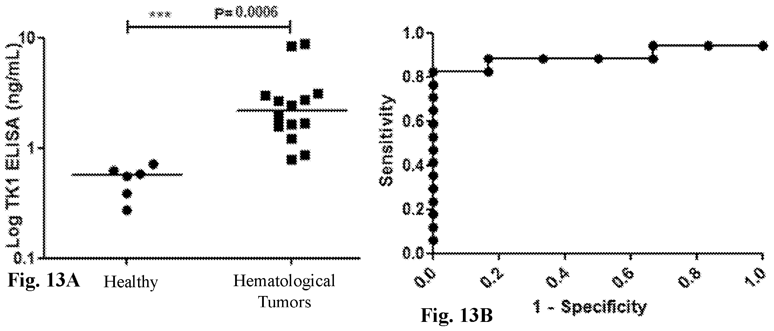

The results from these antibodies are presented in Figs. 12 and 13A-13B. Fig. 12 illustrates a standard curve with different concentrations of recombinant TK1 with CTK1p-211 coating and CTK1p-215 as detection antibodies. Fig. 13A illustrates the log STK1 ELISA protein levels in sera from healthy dogs and dogs with hematological malignancies. Figs. 13B illustrates the corresponding ROC curve for discrimination of hematological tumor dogs from healthy dogs.

The embodiments described above are to be understood as a few illustrative examples of the present invention.

The scope of the present invention is, however, defined by the appended claims.

### REFERENCES

Alegre, M.M., Weyant, M.J., Bennett, D.T., Yu, J.A., Ramsden, M.K., Elnaggar, A., Robison, R., O'Neill, K.L., 2014. Serum detection of thymidine kinase 1 as a means of early detection of lung cancer. Anticancer Research 34, 2145-2152.
Carlsson, L., Larsson, A., Lindman, H., 2009. Elevated levels of thymidine kinase 1 peptide in serum from patients with breast cancer. Uppsala Journal of Medical Sciences 114, 116-120.
Gasparri, F., Wang, N., Skog, S., Galvani, A., Eriksson, S., 2009. Thymidine kinase 1 expression defines an activated G1 state of the cell cycle as revealed with site-specific antibodies and ArrayScan (TM) assays. Eur J Cell Biol 88, 779-785.
Jagarlamudi, KK., Westberg, S., Rbnnberg, H., Eriksson, S., 2014. Properties of cellular and serum forms of thymidine kinase 1 (TK1) in dogs with acute lymphocytic leukemia (ALL) and canine mammary tumors (CMTs): implications for TK1 as a proliferation biomarker. BMC veterinary research, 10:228.
Jagarlamudi, KK., Hansson, LO., Eriksson, S., 2015. Breast and prostate cancer patients differ significantly in their serum Thymidine kinase 1 (TK1) specific activities compared with those hematological malignancies and blood donors: implications of using serum TK1 as a biomarker. BMC Cancer, 15:1073.
Kiran Kumar, J., Sharif, H., Westberg, S., von Euler, H., Eriksson, S., 2013. High levels of inactive thymidine kinase 1 polypeptide in sera from dogs with solid tumours by immunoaffinity methods: Implications for in vitro diagnostics. The Veterinary Journal 197, 854-860.
Kiran Kumar. J., Immunoassays for detection of serum Thymidine kinase 1 in Dog lymphomas and carcinomas, Master Thesis in Animal Sciences, Uppsala 2010*.*
Sharif, H., von Euler, H., Westberg, S., He, E., Wang, L., Eriksson, S., 2012 a. A sensitive and kinetically defined radiochemical assay for canine and human serum thymidine kinase (TK1) to monitor canine lymphoma. The Veterinary Journal 194, 40-47.
Sharif, H., Jagarlamudi, K.K., Wang, L., He, E., Eriksson, S., 2012 b. Quaternary structures of recombinant, cellular, and serum forms of Thymidine Kinase 1 from dogs and humans. BMC Biochemistry 13, 12.
von Euler, H., Ohrvik, A.B., Eriksson, S.K., 2006. A non- radiometric method for measuring serum thymidine kinase activity in malignant lymphomas in dogs. Research in Veterinary Science 80, 17-24.
von Euler, H., Rivera, P., Aronsson, A.C., Bengtsson, C., Hansson, L.O., Eriksson, S.K., 2008. Monitoring therapy in canine malignant lymphoma and leukemia with serum thymidine kinase 1 activity - Evaluation of a new, fully automated non-radiometric assay. International Journal Oncology 34, 505-510.
von Euler, H., Eriksson, S., 2011. Comparative aspects of thymidine kinase 1 in human and canine tumor diseases. Vet Comp Oncol, 9: 1-15.
Wu, C., Yang, R., Zhou, J., Bao, S., Zou, L., Zhang, P., Mao, Y., Wu, J., He, Q., 2003. Production and characterization of a novel chicken IgY antibody raised against C-terminal peptide from human thymidine kinase 1. Journal of Immunological Methods 277, 157-169.

## Claims

1. A kit for determining a level of non-human mammal thymidine kinase 1 (TK1) protein in a sample, comprising:
a first antibody immobilized to a support or intended to be immobilized to said support; and
a second antibody, wherein one of said first antibody and said second antibody has specificity for a peptide consisting of a first amino acid sequence from a C-terminal region of non-human mammal TK1 and the other of said first antibody and said second antibody has specificity for a peptide consisting of an amino acid sequence from an active site of TK1,
wherein said non-human mammal TK1 is selected from a group consisting of canine TK1 protein, feline TK1 protein and equine TK1 protein; and
said other of said first antibody and second antibody has specificity for a peptide consisting of an amino acid sequence of SEQ ID NO: 2, **characterized in that**
said one of said first antibody and said second antibody has specificity for a peptide consisting of an amino acid sequence of SEQ ID NO: 11.

2. The kit according to claim 1, wherein said kit a sandwich assay kit.

3. The kit according to claim 1 or 2, wherein said kit is an Enzyme-Linked Immunosorbent Assay (ELISA) kit.

4. The kit according to any of the claims 1 to 3, wherein said second antibody has a covalently attached biotin or a covalently attached streptavidin or avidin.

5. The kit according to claim 4, further comprising:
a horseradish peroxidase (HRP) labeled streptavidin or avidin or a HRP labeled biotin; and
a HRP substrate selected from a group consisting of a 3,3',5,5'-tetramethylbenzidine (TMB) substrate, a 3,3'-diaminobenzidine (DAB) substrate and a 2,2'-azino-bis(3-ethylbenzothiazoline-6-sulphonic acid (ABTS) substrate.

6. The kit according to any of the claims 1 to 5, further comprising a microtiter plate as said support.

7. The kit according to any of the claims 1 to 5, further comprising agarose beads or magnetic beads as said support.

8. An *in vitro* method for determining a level of canine, feline or equine thymidine kinase 1 (TK1) protein in a sample, comprising:
contacting said sample with a first antibody and a second antibody of said kit according to any of the claims 1 to 8; and
detecting an amount of bound second antibody.

9. The method according to claim 9, further comprising determining said level of canine, feline or equine TK1 protein in said sample based on said detected amount of bound second antibody.

10. An *in vitro* method for estimating the likelihood of recurrence of a tumor disease in a canine, feline or equine subject, comprising:
determining a level of canine, feline or equine thymidine kinase 1 (TK1) protein in a body sample from said canine, feline or equine subject using said kit according to any of the claims 1 to 8 or said *in vitro* method according to claim 9 or 10;
comparing said level of canine, feline or equine TK1 protein in said body sample with a level of canine, feline or equine TK1 protein representative of a population of healthy canine, feline or equine subjects or with a level of canine, feline or equine TK1 protein previously determined in said canine, feline or equine subject; and
estimating said likelihood of recurrence of said tumor disease in said canine, feline or equine subject based on said comparison.

11. An *in vitro* method for determining cell proliferation in a canine, feline or equine subject, comprising:
determining a level of canine, feline or equine thymidine kinase 1 (TK1) protein in a body sample from said canine, feline or equine subject using said kit according to any of the claims 1 to 8 or said *in vitro* method according to claim 9 or 10; and
determining said cell proliferation based on said level of canine, feline or equine TK1 protein in said body sample.

12. An *in vitro* method for determining a proliferation process response in a canine, feline or equine subject suffering from a malignant disease, comprising:
determining a level of canine, feline or equine thymidine kinase 1 (TK1) protein in a body sample from said canine, feline or equine subject using said kit according to any of the claims 1 to 8 or said *in vitro* method according to claim 9 or 10; and
determining said proliferation process response based on said level of canine, feline or equine TK1 protein in said body sample.

13. An *in vitro* method for determining a level of inflammation, infection, or tumor cell proliferation in a canine, feline or equine subject, comprising:
determining a level of canine, feline or equine thymidine kinase (TK1) protein in a body sample from said canine, feline or equine subject using said kit according to any of the claims 1 to 8 or said *in vitro* method according to claim 9 or 10; and
determining said level of inflammation, infection or tumor cell proliferation based on said level of canine, feline or equine TK1 protein in said body sample.

14. An *in vitro* method for evaluating efficiency of treatment of a malignant disease in a canine, feline or equine subject, comprising:
determining a level of canine, feline or equine thymidine kinase 1 (TK1) protein in a body sample from said canine, feline or equine subject using said kit according to any of the claims 1 to 8 or said *in vitro* method according to claim 9 or 10 prior to or in connection with start of said treatment of said malignant disease;
determining a level of canine, feline or equine TK1 protein in a body sample from said canine, feline or equine subject using said kit according to any of the claims 1 to 8 or said *in vitro* method according to claim 9 or 10 during or after said treatment of said malignant disease; and
evaluating efficiency of said treatment of said malignant disease based on a comparison of said level of canine, feline or equine TK1 protein determined in said body sample prior to or in connection with start of said treatment of said malignant disease and said level of canine, feline or equine TK1 protein determined in said body sample during or after said treatment of said malignant disease.

## Patentansprüche

1. Kit zum Bestimmen eines Spiegels von nichtmenschlichem Thymidinkinase-1(TK1)-Protein in einer Probe, Folgendes umfassend:
einen ersten Antikörper, der auf einem Träger immobilisiert ist oder dazu bestimmt ist, auf dem Träger immobilisiert zu werden; und
einen zweiten Antikörper, wobei einer von dem ersten Antikörper und dem zweiten Antikörper eine Spezifität für ein Peptid aufweist, das aus einer ersten Aminosäuresequenz aus einer C-terminalen Region von nichtmenschlicher Säugetier-TK1 besteht, und der andere von dem ersten Antikörper und dem zweiten Antikörper eine Spezifität für ein Peptid aufweist, das aus einer Aminosäuresequenz von einer aktiven Stelle von TK1 besteht,
wobei die nichtmenschliche Säugetier-TK1 ausgewählt ist aus einer Gruppe bestehend aus Hunde-TK1-Protein, Katzen-TK1-Protein und Pferde-TK1-Protein; und
der andere von dem ersten Antikörper und dem zweiten Antikörper eine Spezifität für ein Peptid aufweist, das aus einer Aminosäuresequenz der SEQ ID NO: 2 besteht, **dadurch gekennzeichnet, dass**
der eine von dem ersten Antikörper und dem zweiten Antikörper eine Spezifität für ein Peptid aufweist, das aus einer Aminosäuresequenz der SEQ ID NO: 11 besteht.

2. Kit nach Anspruch 1, wobei der Kit ein Sandwich-Assay-Kit ist.

3. Kit nach Anspruch 1 oder 2, wobei der Kit ein Enzyme-Linked-Immunosorbent-Assay(ELISA)-Kit ist.

4. Kit nach einem der Ansprüche 1 bis 3, wobei der zweite Antikörper ein kovalent gebundenes Biotin oder ein kovalent gebundenes Streptavidin oder Avidin aufweist.

5. Kit nach Anspruch 4, ferner Folgendes umfassend:
ein Meerrettichperoxidase(horseradish peroxidase - HRP)-markiertes Streptavidin oder Avidin oder ein HRP-markiertes Biotin; und
ein HRP-Substrat, ausgewählt aus einer Gruppe bestehend aus einem 3,3',5,5'-Tetramethylbenzidin(TMB)-Substrat, einem 3,3'-Diaminobenzidin(DAB)-Substrat und einem 2,2'-Azino-bis(3-ethylbenzothiazolin-6-sulfonsäure) (ABTS)-Substrat.

6. Kit nach einem der Ansprüche 1 bis 5, ferner umfassend eine Mikrotiterplatte als Träger.

7. Kit nach einem der Ansprüche 1 bis 5, ferner umfassend Agarosekügelchen oder Magnetkügelchen als Träger.

8. *In-vitro*-Verfahren zum Bestimmen eines Spiegels von Hunde-, Katzen- oder Pferde-Thymidinkinase-1(TK1)-Protein in einer Probe, Folgendes umfassend:
Inkontaktbringen der Probe mit einem ersten Antikörper und einem zweiten Antikörper des Kits nach einem der Ansprüche 1 bis 8; und
Nachweisen einer Menge an gebundenem zweitem Antikörper.

9. Verfahren nach Anspruch 9, ferner umfassend das Bestimmen des Spiegels von Hunde-, Katzen- oder Pferde-TK1-Protein in der Probe basierend auf der nachgewiesenen Menge an gebundenem zweitem Antikörper.

10. *In-vitro*-Verfahren zum Schätzen der Wahrscheinlichkeit eines Wiederauftretens einer Tumorerkrankung bei einem Hunde-, Katzenoder Pferde-Subjekt, Folgendes umfassend:
Bestimmen eines Spiegels von Hunde-, Katzen- oder Pferde-Thymidinkinase-1(TK1)-Protein in einer Körperprobe von dem Hunde-, Katzen- oder Pferde-Subjekt unter Verwendung des Kits nach einem der Ansprüche 1 bis 8 oder des *In-vitro*-Verfahrens nach Anspruch 9 oder 10;
Vergleichen des Spiegels von Hunde-, Katzen- oder Pferde-TK1-Protein in der Körperprobe mit einem Spiegel von Hunde-, Katzenoder Pferde-TK1-Protein, das repräsentativ für eine Population von gesunden Hunde-, Katzen- oder Pferde-Subjekten ist, oder mit einem Spiegel von Hunde-, Katzen- oder Pferde-TK1-Protein, das zuvor in dem Hunde-, Katze- oder Pferde-Subjekt bestimmt wurde; und
Schätzen der Wahrscheinlichkeit eines erneuten Auftretens der Tumorerkrankung bei dem Hunde-, Katzen- oder Pferde-Subjekt basierend auf dem Vergleich.

11. *In-vitro*-Verfahren zum Bestimmen der Zellproliferation bei einem Hunde-, Katzen- oder Pferde-Subjekt, Folgendes umfassend:
Bestimmen eines Spiegels von Hunde-, Katzen- oder Pferde-Thymidinkinase-1(TK1)-Protein in einer Körperprobe von dem Hunde-, Katzen- oder Pferde-Subjekt unter Verwendung des Kits nach einem der Ansprüche 1 bis 8 oder des *In-vitro*-Verfahrens nach Anspruch 9 oder 10; und
Bestimmen der Zellproliferation basierend auf dem Spiegel von Hunde-, Katzen- oder Pferde-TK1-Protein in der Körperprobe.

12. *In-vitro*-Verfahren zum Bestimmen einer Proliferationsprozessantwort bei einem an einer malignen Erkrankung leidenden Hunde-, Katzen- oder Pferde-Subjekt, Folgendes umfassend:
Bestimmen eines Spiegels von Hunde-, Katzen- oder Pferde-Thymidinkinase-1(TK1)-Protein in einer Körperprobe von dem Hunde-, Katzen- oder Pferde-Subjekt unter Verwendung des Kits nach einem der Ansprüche 1 bis 8 oder des *In-vitro*-Verfahrens nach Anspruch 9 oder 10; und
Bestimmen der Proliferationsprozessantwort basierend auf dem Spiegel von Hunde-, Katzen- oder Pferde-TK1-Protein in der Körperprobe.

13. *In-vitro*-Verfahren zum Bestimmen eines Grades an Entzündung, Infektion oder Tumorzellproliferation bei einem Hunde-, Katzenoder Pferde-Subjekt, Folgendes umfassend:
Bestimmen eines Spiegels von Hunde-, Katzen- oder Pferde-Thymidinkinase(TK1)-Protein in einer Körperprobe von dem Hunde, Katzen- oder Pferde-Subjekt unter Verwendung des Kits nach einem der Ansprüche 1 bis 8 oder des *In-vitro*-Verfahrens nach Anspruch 9 oder 10; und
Bestimmen des Grades an Entzündung, Infektion oder Tumorzellproliferation basierend auf dem Spiegel von Hunde-, Katzen- oder Pferde-TK1-Protein in der Körperprobe.

14. *In-vitro*-Verfahren zum Bewerten der Wirksamkeit der Behandlung einer malignen Erkrankung bei einem Hunde-, Katzenoder Pferde-Subjekt, Folgendes umfassend:
Bestimmen eines Spiegels von Hunde-, Katzen- oder Pferde-Thymidinkinase-1(TK1)-Protein in einer Körperprobe von dem Hunde-, Katzen- oder Pferde-Subjekt unter Verwendung des Kits nach einem der Ansprüche 1 bis 8 oder des *In-vitro*-Verfahrens nach Anspruch 9 oder 10 vor oder in Verbindung mit dem Beginn der Behandlung der malignen Erkrankung;
Bestimmen eines Spiegels von Hunde-, Katzen- oder Pferde-TK1-Protein in einer Körperprobe von dem Hunde-, Katzen- oder Pferde-Subjekt unter Verwendung des Kits nach einem der Ansprüche 1 bis 8 oder des *In-vitro*-Verfahrens nach Anspruch 9 oder 10 während oder nach der Behandlung der malignen Erkrankung; und
Bewerten der Wirksamkeit der Behandlung der malignen Erkrankung basierend auf einem Vergleich des in der Körperprobe vor oder in Verbindung mit dem Beginn der Behandlung der malignen Erkrankung bestimmten Spiegels von Hunde-, Katzen- oder Pferde-TK1-Protein und des in der Körperprobe während oder nach der Behandlung der malignen Erkrankung bestimmten Spiegels von Hunde-, Katzen- oder Pferde-TK1-Protein.

## Revendications

1. Kit de détermination d'un taux de protéine thymidine kinase 1 (TK1) de mammifère non humain dans un échantillon, comprenant :
un premier anticorps immobilisé sur un support ou destiné à être immobilisé sur ledit support ; et
un second anticorps, dans lequel l'un dudit premier anticorps et dudit second anticorps présente une spécificité pour un peptide constitué d'une première séquence d'acides aminés d'une région C-terminale de TK1 de mammifère non humain et l'autre dudit premier anticorps et dudit second anticorps présente une spécificité pour un peptide constitué d'une séquence d'acides aminés d'un site actif de TK1,
dans lequel ladite TK1 de mammifère non humain est choisie dans un groupe constitué par la protéine TK1 canine, la protéine TK1 féline et la protéine TK1 équine ; et
ledit autre desdits premier anticorps et second anticorps présente une spécificité pour un peptide constitué d'une séquence d'acides aminés de SEQ ID NO : 2, **caractérisé en ce que :**
ledit anticorps parmi ledit premier anticorps et ledit second anticorps présente une spécificité pour un peptide constitué d'une séquence d'acides aminés de SEQ ID NO : 11.

2. Kit selon la revendication 1, dans lequel ledit kit est un kit de dosage en sandwich.

3. Kit selon la revendication 1 ou 2, dans lequel ledit kit est un kit de dosage par immunosorbant lié à une enzyme (ELISA).

4. Kit selon l'une quelconque des revendications 1 à 3, dans lequel ledit second anticorps comprend une biotine liée par covalence ou une streptavidine ou une avidine liée par covalence.

5. Kit selon la revendication 4, comprenant en outre :
une streptavidine ou une avidine marquée par la peroxydase de raifort (HRP) ou une biotine marquée par la HRP ; et
un substrat de HRP choisi dans un groupe constitué d'un substrat 3,3',5,5'-tétraméthylbenzidine (TMB), d'un substrat 3,3'-diaminobenzidine (DAB) et d'un substrat 2,2'-azino-bis (acide 3-éthylbenzothiazoline-6-sulfonique) (ABTS).

6. Kit selon l'une quelconque des revendications 1 à 5, comprenant en outre une plaque de microtitrage en tant que ledit support.

7. Kit selon l'une quelconque des revendications 1 à 5, comprenant en outre des billes d'agarose ou des billes magnétiques en tant que ledit support.

8. Procédé *in vitro* de détermination d'un taux de protéine thymidine kinase 1 (TK1) canine, féline ou équine dans un échantillon, comprenant :
la mise en contact dudit échantillon avec un premier anticorps et un second anticorps dudit kit selon l'une quelconque des revendications 1 à 8 ; et
la détection d'une quantité de second anticorps lié.

9. Procédé selon la revendication 9, comprenant en outre la détermination dudit taux de protéine TK1 canine, féline ou équine dans ledit échantillon sur la base de ladite quantité détectée de second anticorps lié.

10. Procédé *in vitro* d'estimation de la probabilité de récurrence d'une maladie tumorale chez un sujet canin, félin ou équin, comprenant :
la détermination d'un taux de protéine thymidine kinase 1 (TK1) canine, féline ou équine dans un échantillon corporel dudit sujet canin, félin ou équin à l'aide dudit kit selon l'une quelconque des revendications 1 à 8 ou dudit procédé *in vitro* selon la revendication 9 ou 10 ;
la comparaison dudit taux de protéine TK1 canine, féline ou équine dans ledit échantillon corporel avec un taux de protéine TK1 canine, féline ou équine représentatif d'une population de sujets canins, félins ou équins sains ou avec un taux de protéine TK1 canine, féline ou équine préalablement déterminé chez ledit sujet canin, félin ou équin ; et
l'estimation de ladite probabilité de récurrence de ladite maladie tumorale chez ledit sujet canin, félin ou équin sur la base de ladite comparaison.

11. Procédé *in vitro* de détermination de la prolifération cellulaire chez un sujet canin, félin ou équin, comprenant :
la détermination d'un taux de protéine thymidine kinase 1 (TK1) canine, féline ou équine dans un échantillon corporel dudit sujet canin, félin ou équin à l'aide dudit kit selon l'une quelconque des revendications 1 à 8 ou dudit procédé *in vitro* selon la revendication 9 ou 10 ; et
la détermination de ladite prolifération cellulaire sur la base dudit taux de protéine TK1 canine, féline ou équine dans ledit échantillon corporel.

12. Procédé *in vitro* de détermination d'une réponse de processus de prolifération chez un sujet canin, félin ou équin atteint d'une maladie maligne, comprenant :
la détermination d'un taux de protéine thymidine kinase 1 (TK1) canine, féline ou équine dans un échantillon corporel dudit sujet canin, félin ou équin à l'aide dudit kit selon l'une quelconque des revendications 1 à 8 ou dudit procédé *in vitro* selon la revendication 9 ou 10 ; et
la détermination de ladite réponse de processus de prolifération sur la base dudit taux de protéine TK1 canine, féline ou équine dans ledit échantillon corporel.

13. Procédé *in vitro* de détermination d'un niveau d'inflammation, d'infection ou de prolifération de cellules tumorales chez un sujet canin, félin ou équin, comprenant :
la détermination d'un taux de protéine thymidine kinase (TK1) canine, féline ou équine dans un échantillon corporel dudit sujet canin, félin ou équin à l'aide dudit kit selon l'une quelconque des revendications 1 à 8 ou dudit procédé *in vitro* selon la revendication 9 ou 10 ; et
la détermination dudit niveau d'inflammation, d'infection ou de prolifération de cellules tumorales sur la base dudit taux de protéine TK1 canine, féline ou équine dans ledit échantillon corporel.

14. Procédé *in vitro* d'évaluation de l'efficacité de traitement d'une maladie maligne chez un sujet canin, félin ou équin, comprenant :
la détermination d'un taux de protéine thymidine kinase 1 (TK1) canine, féline ou équine dans un échantillon corporel dudit sujet canin, félin ou équin à l'aide dudit kit selon l'une quelconque des revendications 1 à 8 ou dudit procédé *in vitro* selon la revendication 9 ou 10 avant ou en liaison avec le début dudit traitement de ladite maladie maligne ;
la détermination d'un taux de protéine TK1 canine, féline ou équine dans un échantillon corporel dudit sujet canin, félin ou équin à l'aide dudit kit selon l'une quelconque des revendications 1 à 8 ou dudit procédé *in vitro* selon la revendication 9 ou 10 pendant ou après ledit traitement de ladite maladie maligne ; et
l'évaluation de l'efficacité dudit traitement de ladite maladie maligne sur la base d'une comparaison dudit taux de protéine TK1 canine, féline ou équine déterminé dans ledit échantillon corporel avant ou en liaison avec le début dudit traitement de ladite maladie maligne et dudit taux de protéine TK1 canine, féline ou équine déterminé dans ledit échantillon corporel pendant ou après ledit traitement de ladite maladie maligne.
